# EUROPEAN PATENT APPLICATION

(11) **EP 4 282 438 A1**
(43) Date of publication of application: **29.11.2023**
(21) Application number: 22174909.6
(22) Date of filing: 23.05.2022
(51) Int. Cl.: A61K 51/04, A61K 103/10, A61P 35/00

(54) **FUNCTIONALIZED BISAMINOTHIOL DERIVATIVES, COMPLEXES WITH THESE BISAMINOTHIOL DERIVATIVES AND USE OF SAID COMPLEXES AS DIAGNOSTICS AND THERAPEUTICS**

(71) Applicant: ABX Advanced Biochemical Compounds GmbH, 01454 Radeberg (DE); Helmholtz-Zentrum Dresden - Rossendorf e.V., 01328 Dresden (DE)
(72) Inventor: LIS, Christian, 01309 Dresden (DE); HOEPPING, Alexander, 01324 Dresden (DE); LANKAU, Hans-Joachim, 01689 Weinböhla (DE); MEYER, Christoph, 01454 Radeberg (DE); JOSEPH, Desna, Albuquerque, New Mexico 87102 (US); SCHULTZE, Christiane, 01307 Dresden (DE); FISCHER, Steffen, 01328 Dresden (DE); LUDWIG, Friedrich-Alexander, 01328 Dresden (DE); ULLRICH, Martin, 01328 Dresden (DE); SIHVER, Wiebke, 01328 Dresden (DE)
(74) Representative: Riechelmann & Carlsohn Patentanwälte PartG mbB

(57) **Abstract**

The invention relates to a compound of general formula I 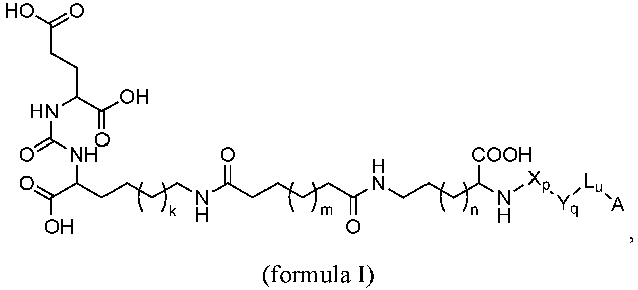 wherein
A is a chelator selected from the group consisting of 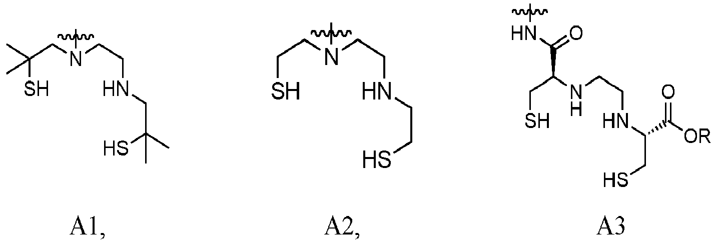 and 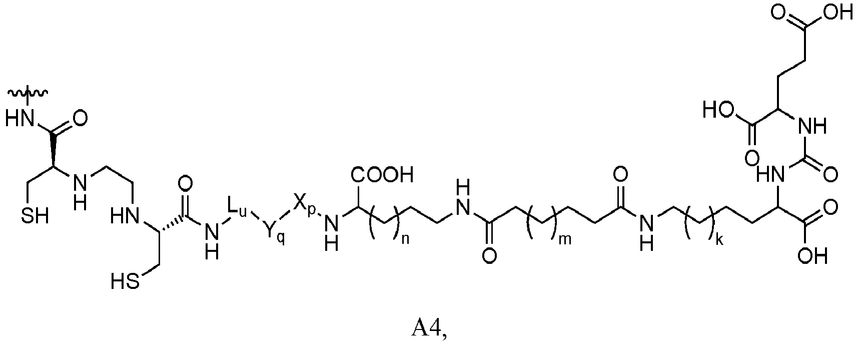 k is independently at each occurrence 0, 1, or 2;
m is independently at each occurrence 1, 2, 3, 4 or 5;
n is independently at each occurrence 0, 1, 2 or 3;
p is independently at each occurrence 1, 2 or 3;
q is independently at each occurrence 1, 2 or 3;
u is independently at each occurrence 0 or 1;
X and Y are substituted or unsubstituted amino acids;
L is a bifunctional linker selected from group, consisting of 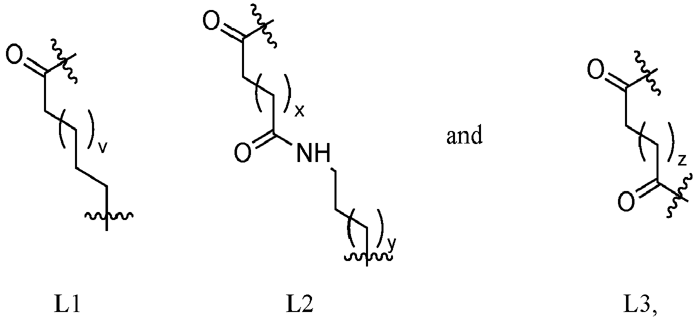 wherein v, x, and y are independently of each other 0, 1, 2, or 3 and z is 0, 1, 2 ,3, 4 or 5; and
R is H, methyl or ethyl.

## Description

The invention relates to functionalized bisaminothiol derivatives. Furthermore, it relates to complexes of these functionalized bisaminothiol derivatives with metals, in particular with radio-metals. In addition, it relates to the use of said complexes, in particular their use in the diagnosis and treatment of diseases in which the prostate-specific membrane antigen (PSMA) is involved.

In personal medicine, radiolabeled agents that are used to target disease-specific biological structures for diagnosis and subsequent therapy of that disease are called theranostics. This approach has been used to locate and subsequently eliminate metastatic disease.

Compared to normal prostate epithelial cells the expression of prostate specific membrane antigen (PSMA) is increased in human prostate cancers and metastatic malignancies thereof. This high and specific expression of PSMA on the cell surface is a feature of localized as well as metastatic prostate cancer. Furthermore, PSMA expression was observed in the neovasculature of multiple non-prostatic solid malignancies (Chang et al., Clin Cancer Res 1999, 5, 2674).

Therefore, PSMA is an attractive target for diagnosis, staging, detection of recurrence and treatment of prostate cancer (and other diseases where PSMA is upregulated) using radiolabeled compounds. Such molecules typically comprise a pharmacophore for PSMA-binding, a linker structure for the optimization of the pharmacological properties and a chelator or prosthetic group to incorporate an appropriate radionuclide. In the recent years mainly ¹⁸F and ⁶⁸Ga labeled radioligands have been used for PET imaging of prostate cancer including ⁶⁸Ga-PSMA-11, ¹⁸F-DCFPyL and ¹⁸F-PSMA-1007.

^{99m}Tc is a popular isotope in nuclear medicine for SPECT imaging due to its longer half-life (t_{1/2} = 6.01 h), low radiotoxicity as well as the widespread and non-centralized availability from ⁹⁹Mo/^{99m}Tc-generators. Due to similar coordination chemistries technetium and rhenium represent a theranostic pair allowing the use of the same PSMA-ligand for SPECT imaging when labeled with ^{99m}Tc and for radioligand therapy (RLT) when labeled with ¹⁸⁶Re (t_{1/2} = 89.25h h) or ¹⁸⁸Re (t_{1/2} = 17.02 h).

Early ^{99m}Tc-labeled PSMA-ligands suffered from slow distribution, high liver uptake and slow clearance from the body. Slow pharmacokinetic properties of ^{99m}Tc- labeled tracers require late imaging timepoints to achieve sufficient tumor-to-background ratios (Werner et al., EJNMMI Research 2020, 10, 45; Vallabhajosula et al., J Nucl Med 2014, 55, 1791). This results in a longer waiting time for the patient between injection of the tracer and acquisition of the SPECT image. In case of a therapeutic application a slow clearance can result in high uptake in off-target organs and hence adverse effects due to radiotoxicity.

Known PSMA-ligands with HYNIC chelating moieties require co-ligands in order to stabilize the technetium complexes in biological systems and to prevent oxidation by oxygen. The choice of the co-ligand can influence the complex geometry, the number of PSMA-ligands coordinated to one Tc-atom and ultimately the biodistribution of the radiolabeled compound. The hydrazine-moiety of HYNIC is a strong nucleophile, which can lead to undesired side products during radiolabeling or when applied in biological systems. The formation of stable complexes of rhenium with HYNIC as the chelator remains challenging (North et al., Inorg. Chem. 2017, 56, 9725; Philip J. Blower, Int. J. Nucl. Medi. Res. 2017, 39).

In conclusion ^{99m}Tc-labeled PSMA-ligands for SPECT imaging of the prior art are inferior to current PSMA-ligands labeled with PET-nuclides in terms of radiolabeling, stability, pharmacokinetics and diagnostic performance. Hence it is desirable to develop new ^{99m}Tc-labeled PSMA-Ligands with optimized pharmacological properties while using a chelator that can form also stable complexes with other metals such as rhenium.

The problem of the invention is to eliminate the drawbacks according to the prior art. In particular, functionalized bisaminothiol derivatives are to be provided that allow formation of stable complexes with metals, in particular with rhenium and technetium. In addition, complexes are to be provided that serve as ligands for the prostate-specific membrane antigen (PSMA) and can be used in diagnosis and treatment of certain diseases in which PSMA is involved.

This problem is solved by the features of claims 1, 8, 10, 11, 13 and 14. Practical developments of the inventions result from the features of the dependent claims.

According to the invention there is provided a compound of general formula I 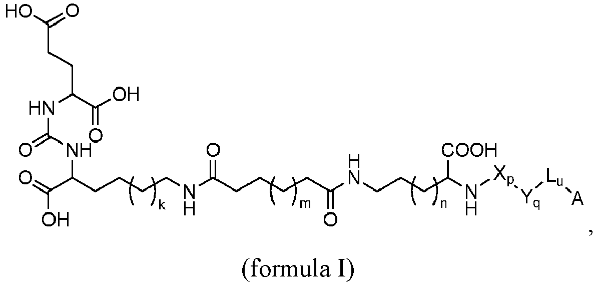 wherein
A is a chelator selected from the group consisting of 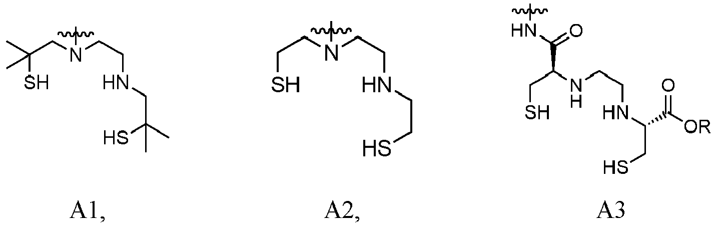 and 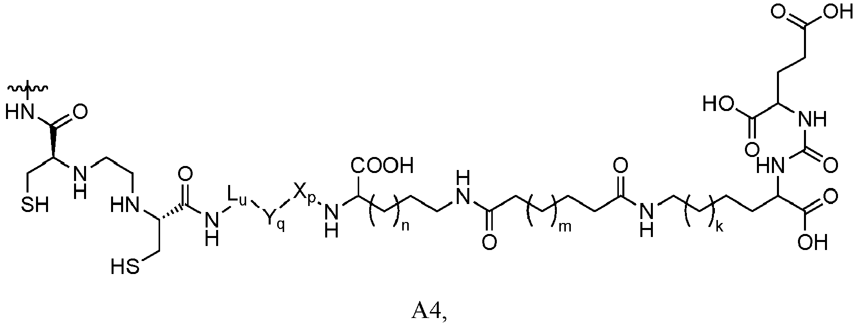
k is independently at each occurrence 0, 1, or 2;
m is independently at each occurrence 1, 2, 3, 4 or 5;
n is independently at each occurrence 0, 1, 2 or 3;
p is independently at each occurrence 1, 2 or 3;
q is independently at each occurrence 1, 2 or 3;
u is independently at each occurrence 0 or 1;
X and Y are substituted or unsubstituted amino acids;
L is a bifunctional linker selected from group, consisting of 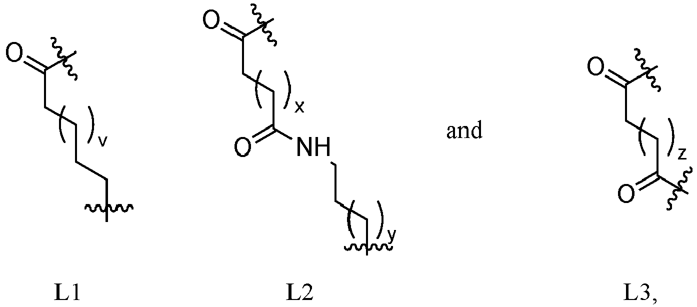 wherein v, x, and y are independently of each other 0, 1, 2, or 3 and z is 0, 1, 2 ,3, 4 or 5; and
R is H, methyl or ethyl.

The compounds of general formula I are bisaminothiol derivates because they contain group A. In the following, group A is also referred to as N₂S₂ chelator. The compounds of general formula I can form complexes with metals, in particular with radiometals. The bisaminothiol moiety of a compound of general formula I allows complexation of a metal, in particular a radiometal, whereby complexes are obtained. Due to the bisaminothiol moiety a compound of general formula I is a ligand for complexation of metals, in particular radiometals.

The compounds of general formula I are functionalized bisaminothiol derivates because they are functionalized by different amino acid sequences as a linker and a urea-based pharmacophore group.

The complexes can be used as ligands that bind to the prostate-specific membrane antigen (PSMA). Namely, it has been found that the complexes can be used as PSMA inhibitors. Thus, the complexes can be used in the diagnosis and treatment of diseases in which PSMA is involved. In particular, the complexes can be used in the diagnosis and treatment of certain diseases where PSMA is upregulated. Thus, the complexes can be used as medicaments in prostate cancer diagnosis and therapy.

According to the invention group X is an amino acid (p = 1) or a sequence of amino acids (p = 2 or 3). If p is 1, then preferably the amino acid is selected from the group consisting of a substituted or unsubstituted glutamic acid, substituted or unsubstituted aspartic acid, substituted or unsubstituted phenylalanine, substituted or unsubstituted histidine and substituted or unsubstituted serine. Preferably, the amino acid is substituted or unsubstituted phenylalanine or substituted or unsubstituted glutamic acid, particularly preferred substituted or unsubstituted glutamic acid. If p is 2 or 3, then preferably each of the amino acids forming the sequence of amino acids independently is selected from the group consisting of a substituted or unsubstituted glutamic acid, substituted or unsubstituted aspartic acid, substituted or unsubstituted phenylalanine, substituted or unsubstituted histidine and substituted or unsubstituted serine. Preferably, each of the amino acids of the sequence of amino acids is substituted or unsubstituted phenylalanine or substituted or unsubstituted glutamic acid, particularly preferred substituted or unsubstituted glutamic acid.

It may be provided that group X is attached to group Y via a first peptide bond and to the moiety BM via a second peptide bond. Moiety BM is a moiety of general formula 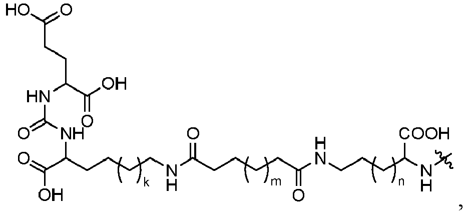 wherein k, m, and n have the meanings given in context with general formula I. Thus, the compound of general formula I can also be represented by general formula

BM-Xₚ-Y_{q}-Lᵤ-A.

If group X has a sequence of two or three amino acids the amino acids are attached to each by a peptide bond. Group BM is a urea-based pharmacophore group. Groups X and Y form a linker between group BM on the one hand and moiety L-A on the other hand.

According to the invention, group Y is an amino acid (q = 1) or a sequence of amino acids (q = 2 or 3). If q is 1, then preferably the amino acid is selected from the group consisting of substituted or unsubstituted glutamic acid, substituted or unsubstituted aspartic acid, substituted or unsubstituted phenylalanine, substituted or unsubstituted histidine and substituted or unsubstituted serine. Preferably, the amino acid is substituted or unsubstituted phenylalanine or substituted or unsubstituted glutamic acid, particularly preferred substituted or unsubstituted glutamic acid. If q is 2 or 3, then preferably each of the amino acids forming the sequence of amino acids independently is selected from the group consisting of a substituted or unsubstituted glutamic acid, substituted or unsubstituted aspartic acid, substituted or unsubstituted phenylalanine, substituted or unsubstituted histidine and substituted or unsubstituted serine. Preferably, each of the amino acids of the sequence of amino acids is substituted or unsubstituted phenylalanine or substituted or unsubstituted glutamic acid, particularly preferred substituted or unsubstituted glutamic acid.

It may be provided that group Y is attached to group X via a first peptide bond and to moiety Lᵤ via a second peptide bond. If group X has a sequence of two or three amino acids the amino acids are attached to each other by a peptide bond.

Groups X and Y can be the same or different. In one embodiment of the invention, the term "substituted amino acid" relates to an amino acid having a phenyl ring, wherein the phenyl ring has one or two substituents independently selected from the group consisting of halogen and hydroxy. The term "halogen", unless stated otherwise, refers to fluorine, chlorine, bromine, or iodine. Preferably, the halogen is iodine. In a preferred embodiment, the phenyl ring has two substituents, wherein one of the substituents is iodine and the other substituent is hydroxy.

According to the invention it may be provided that L in the compounds of formula I is L1 or L2, wherein v, x, and y are independently of each other 1, 2, or 3. In a more preferred embodiment of the invention the linker L may be L1 or L2, wherein v, x, and y are independently of each other 1. In a more preferred embodiment of the invention the linker L is L1, wherein v is 1.

According to the invention it may be provided that A in the compounds of formula I may be A1 or A2. In a more preferred embodiment of the invention A is A1.

In a more preferred embodiment according to the invention compounds of general formula I may be provided, wherein k is 1, m is 3, n is 2, p is 1 or 2, q is 1 or 2 and u is 1, preferably p and q are 1, X and Y independently are substituted or unsubstituted phenylalanine or glutamic acid; L is L1 with v = 1 or L2 with x and y = 1, and A is A1 or A2.

In a first embodiment of the invention, X is unsubstituted phenylalanine and Y is substituted or unsubstituted phenylalanine. Preferably, X is unsubstituted phenylalanine and Y is substituted phenylalanine, more preferably X is unsubstituted phenylalanine and Y is phenylalanine substituted with iodine and OH at the phenyl ring. In the first embodiment, it is preferred that L is L1 or L2, particularly preferred L1. Preferably, L is L1 with v = 1 or L2 with x and y = 1. In the first embodiment, it is preferred that A is selected form the group consisting of A1 to A4. A is preferably A1 or A2, particularly preferred A1. In the first embodiment, it is preferred that k is 1, m is 3, n is 2, p is 1, q is 1 and u is 1.

In a second embodiment of the invention, X and Y are independently of each other phenylalanine or glutamic acid. Preferably, X and Y are both glutamic acid. In the second embodiment, it is preferred that L is L1 or L2, particularly preferred L1. Preferably, L is L1 with v = 1 or L2 with x and y = 1. In the first embodiment, it is preferred that A is selected form the group consisting of A 1 bis A4. A preferably is A1 or A2, particularly preferred A1. In the first embodiment, it is preferred that k is 1, m is 3, n is 2, p is 1, q is 1 and u is 1.

If the moiety A is A4, it is preferred that k, m, n, p, q, v, x, y and z as well as X, Y and L in A4 have the same meaning as in moiety BM.

According to the invention the following compounds 1, 2, 4, 5, and 6 are preferred. 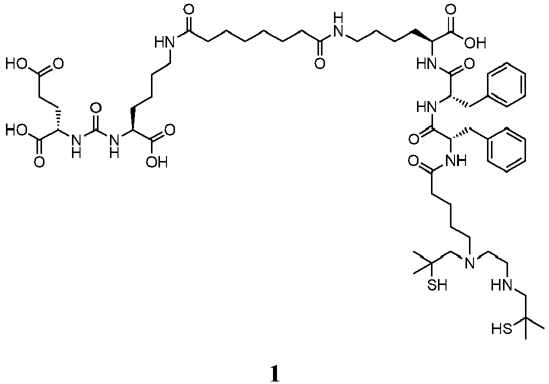 Compound 1 is a compound of general formula I, wherein k is 1, m is 3, n is 2, p, q and u are 1, X and Y are phenylalanine; L is L1 with v = 1 and A is A1. 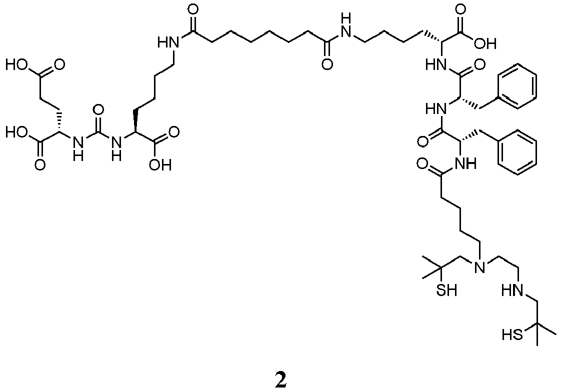 Compound 2 is a compound of general formula I, wherein k is 1, m is 3, n is 2, p, q and u are 1, X and Y are phenylalanine; L is L1 with v = 1 and A is A1. 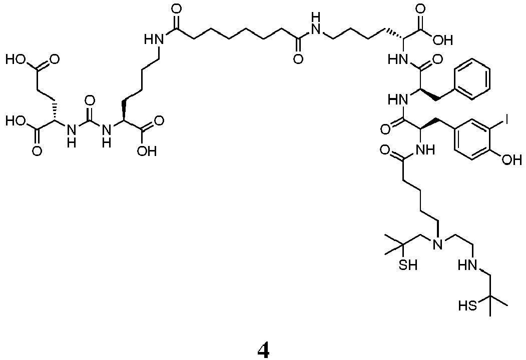 Compound 4 is a compound of general formula I, wherein k is 1, m is 3, n is 2, p, q and u are 1, X is unsubstituted phenylalanine and Y is phenylalanine substituted with iodine and OH at the phenyl ring; L is L1 with v = 1 and A is A1. 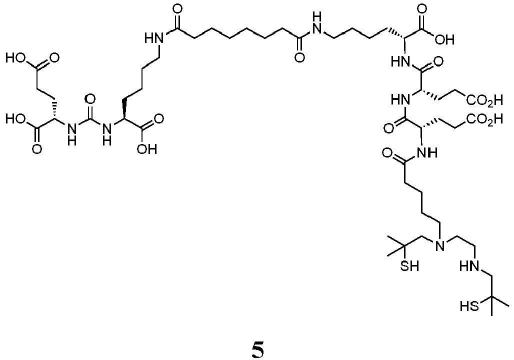 Compound 5 is a compound of general formula I, wherein k is 1, m is 3, n is 2, p, q and u are 1, X and Y are glutamic acid; L is L1 with v = 1 and A is A1. 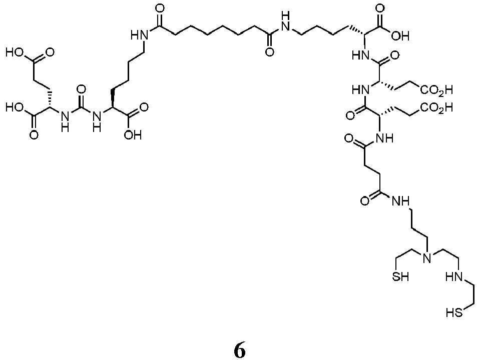 The compound (6) is a compound of general formula I, wherein k is 1, m is 3, n is 2, p, q and u are 1, X and Y are glutamic acid; L is L2 with x and y = 1, and A is A2.

The compounds of general formula I may, depending on their structure, exist in tautomeric or stereoisomeric forms. Thus, compounds of general formula I comprise all enantiomers as well as all diastereomers. The invention therefore also encompasses the tautomers, enantiomers, or diastereomers and respective mixtures of a compound of general formula I. The stereoisomerically uniform constituents can be isolated in a known manner from such mixtures of enantiomers and/or diastereomers.

According to the invention there is further provided a complex comprising a compound of general formula I as ligands and a metal. Such a complex can be used as a medicinal drug, in particular a medicinal drug for diagnosis and therapy of diseases of the prostate. Complexation of the metal preferably takes place via the chelator moiety A. The complexes are coordination complexes of the corresponding metal. The metal can be present as an ion of the metal or as an oxide of the metal. In the metal oxide, the metal can be present as an ion. In one embodiment, the complex may consist of the compound of general formula I as ligands and the metal ion. In another embodiment, the complex may consist of the compound of general formula I as ligands and the metal oxide. The metal forms the central particle of the inventive complex.

In a preferred embodiment of the invention the metal is selected from the group consisting of a rhenium ion, a technetium ion, and a copper ion. Preferably, the metal is a radioactive metal. Thus, a preferred complex is a metal complex comprising a radionuclide and a compound of general formula I.

More preferably, the metal is an isotope selected from the group consisting of ^{99m}Tc, ⁹⁹Tc, ^{94m}Tc, ¹⁸⁶Re, ¹⁸⁸Re, ⁶⁴Cu and ⁶⁷Cu. Even more preferably, the metal is an isotope selected from the group consisting of ^{99m}Tc, ¹⁸⁶Re and ¹⁸⁸Re. In a most preferred embodiment of the invention the isotope is ^{99m}Tc.

In one embodiment of the invention the complex is a compound of formula ReO-5: 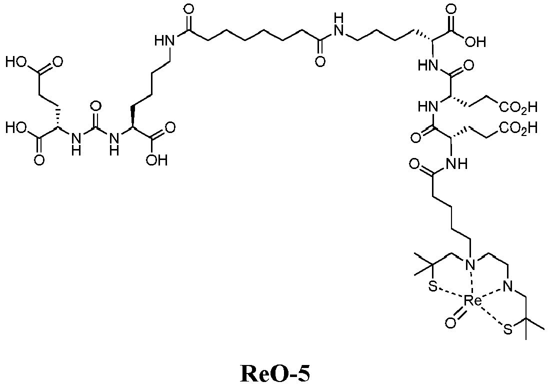 wherein Re is a rhenium ion. Preferably, the rhenium ion is ¹⁸⁶Re or ¹⁸⁸Re. The rhenium ion is present as a part of ReO.

In one embodiment of the invention the complex is a compound of formula TcO-5: 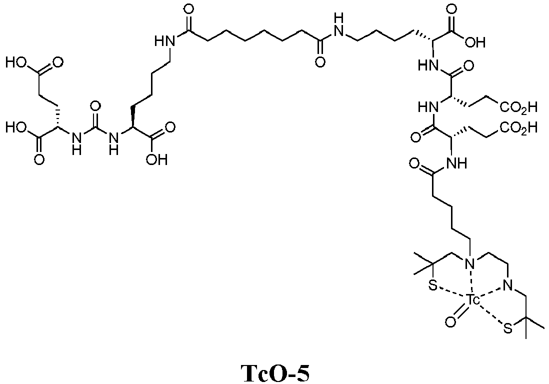 wherein Tc is a technetium ion. Preferably, the technetium ion is an isotope selected from the group consisting of ^{99m}Tc, ⁹⁹Tc, ^{94m}Tc. The technetium ion is present as a part of TcO. Especially preferred, the technetium ion is the isotope ^{99m}Tc. In a preferred embodiment of the invention the complex is a compound of formula [^{99m}Tc]TcO-5:

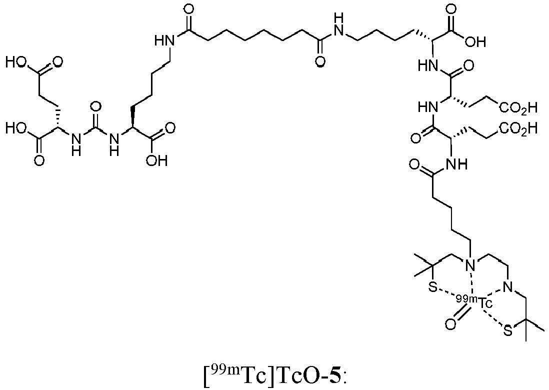

The formation of the ^{99m}Tc-containing complexes and their supply for application can be achieved by reaction of the respective ligand, i.e. the respective compound of general formula I, with a solution of sodium [^{99m}Tc]pertechnetate in saline, as provided by commercial available ⁹⁹Mo/^{99m}Tc generators, on the basis of methods described in the literature (IAEA. *Labelling of small biomolecules using novel Technetium-99m Cores;* International Atomic Energy Agency: Vienna, 2007.), which were applied and adapted. To enable the necessary reduction of ^{99m}Tc(VII), present in sodium [^{99m}Tc]pertechnetate, to ^{99m}Tc(V), present as ^{99m}Tc(V)O in the complex, a reducing agent, preferably stannous chloride is used under optimized conditions, such as its preparation under acidic conditions, preferably using diluted hydrochloric acid under saturation by a stream of an inert gas, e.g. helium, and therefore exclusion of oxygen. The labelling step is performed under weakly acidic conditions, such as a pH range of 5 to 6.5. The overall volume of the reaction solution can be in the range of 0.2-12 mL and the amount of sodium (^{99m}Tc)pertechnetate can be in the range 0.1-50 GBq. Depending on the volume of generator eluate used, the amount of used ligand may vary (10-200 µg). The labelling step can be conducted at room temperature. Reaction parameters used in particular might require elevated temperatures of up to 100°C, preferably 80°C, to complete the reaction or minimize reaction time. Furthermore, heating for a certain period of time, e.g. for 20 min at 80°C, is of advantage due to resulting decomposition of unreacted amounts of excess ligand and therefore supporting imaging properties of the complex. In case of incomplete labelling the complex containing solution can be purified by means of solid phase extraction (SPE) procedures or semi-preparative HPLC, e.g. using RP-C18 or Sephadex (G-25, superfine) as stationary phases. Auxiliary materials contributing to complete conversion and minimizing reaction time, such as salts of gluconic or heptagluconic acid, preferably calcium heptagluconate, can be used. Other auxiliary materials, such as D-mannose, galactose or cyclodextrins, can be used, of which D-mannose is preferred. In the same manner, the use of stabilizing and antioxidative agents, such as dithiothreitol and ascorbic acid, respectively, is possible.

The complexes according to the invention can bind to the prostate-specific membrane antigen (PSMA). Thus, they are ligands for the prostate-specific membrane antigen (PSMA). In the following, the complexes according to the invention are also referred to as ligands or PSMA ligands. If the complex has a radioactive metal, then the complex is also referred to as radioligand or PSMA radioligand.

The PSMA ligands according to the invention comprise the urea-based pharmacophore group BM, different amino acid sequences in the linker -X-Y- and different N₂S₂ chelators. A synthesis strategy which requires no mercury-containing intermediates was used, allowing the preparation of the inventive PSMA ligands free of potentially toxic mercury impurities. Mercury salts are commonly applied to cleave thiol-protecting groups; see Peter G.M. Wuts, Greene's Protective Groups in Organic Synthesis, Fifth Edition, Wiley, 2014).

The compounds of general formula I may be synthesized in different ways by means of known synthesis pathways, for example by use of the Merrifield synthesis (see Robert Bruce Merrifield, Solid phase peptide synthesis, Journal of the American Chemical Society, Volume 85, issue 14 p. 2149-2154). In one way, there is provided a compound of general formula II 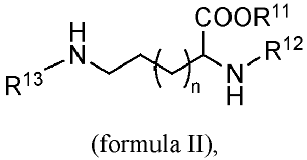 wherein R¹¹ represents a solid support, preferably 2-CTC resin, R¹² and R¹³ represent an orthogonal protecting groups and n has the meanings given in context with general formula I. In a preferred embodiment R¹² is Fmoc and R¹³ is Dde. In another preferred embodiment or R¹² is Dde and R¹³ is Fmoc.

If R¹² is Dde and R¹³ is Fmoc, a first synthesis route can be used. In the first synthesis route, there is performed an Fmoc deprotection of the compound of general formula II in a first step whereby a first reaction product of general formula III 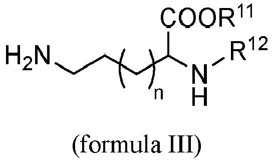 is obtained. In a second step, an amide coupling between the first reaction product of general formula III and a compound of general formula IV 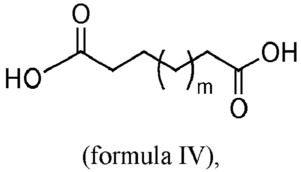 is performed, whereby a second reaction product of general formula V 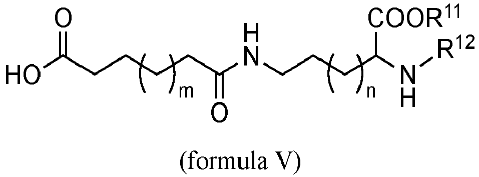 is obtained. In a third step, an amide coupling between the second reaction product of general formula V and a compound of general formula VI 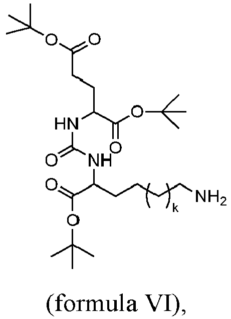 is performed, whereby a third reaction product of general formula VII 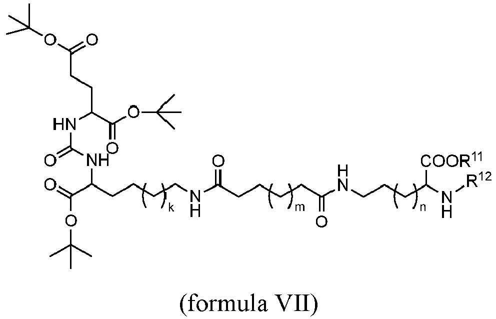 is obtained. In a fourth step, a Dde cleavage of the compound of general formula VII is performed whereby a fourth reaction product of general formula VIII 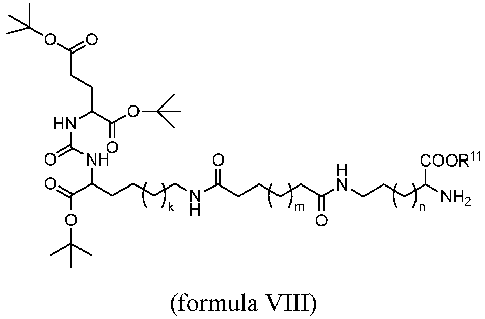 is obtained.

In a fifth step, a fifth reaction product of general formula IX 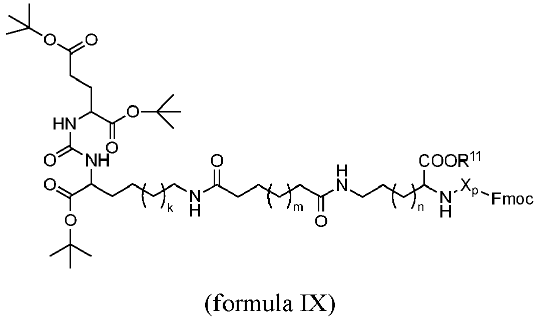 is synthesized. The moiety Xₚ corresponds to the moiety Xₚ of general formula I with the provision that reactive side chains can be protected by protecting groups. For example, -COOH may be protected as -COOtBu. To synthesize a reaction product of general formula IX, an amide coupling between the fourth reaction product of general formula VIII and a compound of general formula Fmoc-X-OH is performed, if p = 1. If p = 2, a first amide coupling between the fourth reaction product of general formula VIII and a first compound of general formula Fmoc-X-OH is performed, whereby an intermediate is obtained, and, after Fmoc deprotection of the intermediate, a second amide coupling between the Fmoc deprotected intermediate and a second compound of general formula Fmoc-X-OH is performed. If p = 3, a first amide coupling between the fourth reaction product of general formula VIII and a first compound of general formula Fmoc-X-OH is performed, whereby a first intermediate is obtained, and, after Fmoc deprotection of the first intermediate, a second amide coupling between the Fmoc deprotected first intermediate and a second compound of general formula Fmoc-X-OH is performed, whereby a second intermediate is obtained, and, after Fmoc deprotection of the second intermediate, a third amide coupling between the Fmoc deprotected second intermediate and a third compound of general formula Fmoc-X-OH is performed.

In a sixth step, a sixth reaction product of general formula X 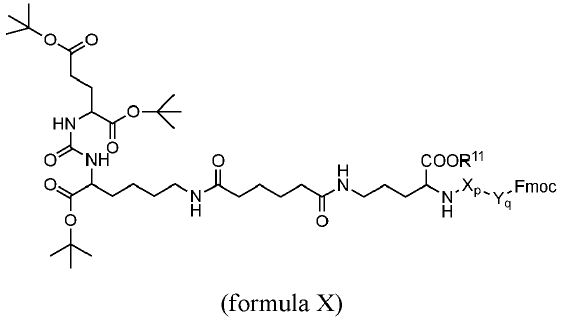 is synthesized. The moiety Y_{q} corresponds to the moiety Y_{q} of general formula I with the provision that reactive side chains can be protected by protecting groups. For example, -COOH may be protected as -COOtBu. To synthesize a reaction product of general formula X, a Fmoc deprotection of the reaction product of general formula IX and then an amide coupling between the Fmoc deprotected reaction product of general formula IX and a compound of general formula Fmoc-Y-OH is performed, if q = 1. If q = 2, a Fmoc deprotection of the reaction product of general formula IX and then a first amide coupling between Fmoc deprotected reaction product of general formula IX and a first compound of general formula Fmoc-Y-OH is performed, whereby an intermediate is obtained, and, after Fmoc deprotection of the intermediate, a second amide coupling between the Fmoc deprotected intermediate and a second compound of general formula Fmoc-Y-OH is performed. If q = 3, a Fmoc deprotection of the reaction product of general formula IX and then a first amide coupling between Fmoc deprotected reaction product of general formula IX and a first compound of general formula Fmoc-Y-OH is performed, whereby a first intermediate is obtained, and, after Fmoc deprotection of the first intermediate, a second amide coupling between the Fmoc deprotected first intermediate and a second compound of general formula Fmoc-Y-OH is performed, whereby a second intermediate is obtained, and, after Fmoc deprotection of the second intermediate, a third amide coupling between the Fmoc deprotected second intermediate and a third compound of general formula Fmoc-Y-OH is performed.

If u = 1, in a seventh step, an Fmoc deprotection of the reaction product of general formula X and then an amide coupling between the obtained reaction product and a compound of general formula Fmoc-L-OH is performed, whereby a reaction product of general formula XI 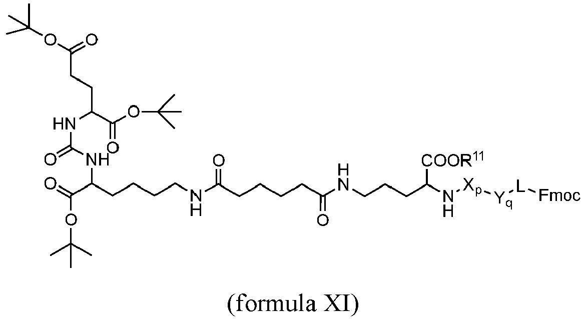 is obtained. The moiety L corresponds to the moiety L of general formula I with the provision that reactive side chains can be protected by protecting groups.

In an eighth step, an Fmoc deprotection of the reaction product of general formula XI, if u = 1, or the reaction product of general formula X, if u = 0, and then an amide coupling between the obtained reaction product and a compound of general formula A-OH is performed, whereby a reaction product of general formula XII 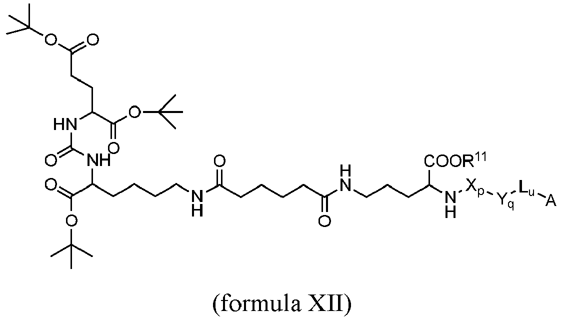 is obtained. The moiety A corresponds to the moiety A of general formula I with the provision that reactive side chains can be protected by protecting groups. For example, -COOH may be protected as -COOtBu and PMB-protecting groups may be on the thiol-groups of A. In a next step, the reaction product of general formula XII is cleaved from the 2-CTC resin and deprotected by removing all protective groups whereby the compound of general formula I is obtained. In formulae II to XII, the indices k, m, n, p, q, u, v, x, y and z have the meanings given in the context of general formula I.

If R¹² is Fmoc and R¹³ is Dde, a second synthesis route can be used. In the second synthesis route, there is performed an Fmoc deprotection of the compound of general formula II in a first step whereby a first reaction product of general formul XIII 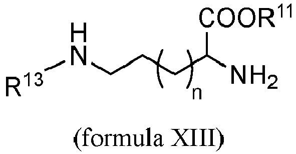 is obtained. In a second step, a second reaction product of general formula XIV 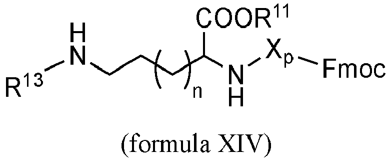 is synthesized. The moiety Xₚ corresponds to the moiety Xₚ of general formula I with the provision that reactive side chains can be protected by protecting groups. For example, -COOH may be protected as -COOtBu. To synthesize a reaction product of general formula XIV, an amide coupling between the first reaction product of general formula XIII and a compound of general formula Fmoc-X-OH is performed, if p = 1. If p = 2, a first amide coupling between the first reaction product of general formula XIII and a first compound of general formula Fmoc-X-OH is performed, whereby an intermediate is obtained, and, after Fmoc deprotection of the intermediate, a second amide coupling between the Fmoc deprotected intermediate and a second compound of general formula Fmoc-X-OH is performed. If p = 3, a first amide coupling between the first reaction product of general formula XIII and a first compound of general formula Fmoc-X-OH is performed, whereby a first intermediate is obtained, and, after Fmoc deprotection of the first intermediate, a second amide coupling between the Fmoc deprotected first intermediate and a second compound of general formula Fmoc-X-OH is performed, whereby a second intermediate is obtained, and, after Fmoc deprotection of the second intermediate, a third amide coupling between the Fmoc deprotected second intermediate and a third compound of general formula Fmoc-X-OH is performed.

In a third step, a third reaction product of general formula XV 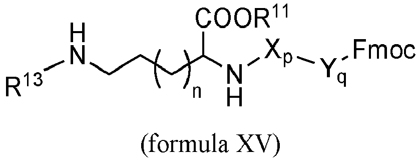 is synthesized. The moiety Y_{q} corresponds to the moiety Y_{q} of general formula I with the provision that reactive side chains can be protected by protecting groups. For example, -COOH may be protected as -COOtBu. To synthesize a reaction product of general formula XV, a Fmoc deprotection of the reaction product of general formula XIV and then an amide coupling between the Fmoc deprotected reaction product of general formula XIV and a compound of general formula Fmoc-Y-OH is performed, if q = 1. If q = 2, a Fmoc deprotection of the reaction product of general formula XIV and then a first amide coupling between Fmoc deprotected reaction product of general formula XIV and a first compound of general formula Fmoc-Y-OH is performed, whereby an intermediate is obtained, and, after Fmoc deprotection of the intermediate, a second amide coupling between the Fmoc deprotected intermediate and a second compound of general formula Fmoc-Y-OH is performed. If q = 3, a Fmoc deprotection of the reaction product of general formula XIV and then a first amide coupling between Fmoc deprotected reaction product of general formula XIV and a first compound of general formula Fmoc-Y-OH is performed, whereby a first intermediate is obtained, and, after Fmoc deprotection of the first intermediate, a second amide coupling between the Fmoc deprotected first intermediate and a second compound of general formula Fmoc-Y-OH is performed, whereby a second intermediate is obtained, and, after Fmoc deprotection of the second intermediate, a third amide coupling between the Fmoc deprotected second intermediate and a third compound of general formula Fmoc-Y-OH is performed.

If u = 1, in a fourth step, an Fmoc deprotection of the reaction product of general formula XV and then an amide coupling between the obtained reaction product and a compound of general formula Fmoc-L-OH is performed, whereby a reaction product of general formula XVI 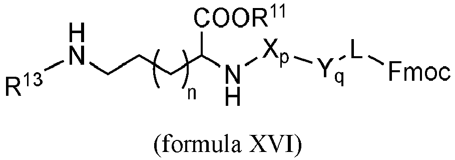 is obtained. The moiety L corresponds to the moiety L of general formula I with the provision that reactive side chains can be protected by protecting groups.

In a fifth step, an Fmoc deprotection of the reaction product of general formula XVI, if u = 1, or the reaction product of general formula XV, if u = 0, and then an amide coupling between the obtained reaction product and a compound of general formula A-OH is performed, whereby a reaction product of general formula XVII 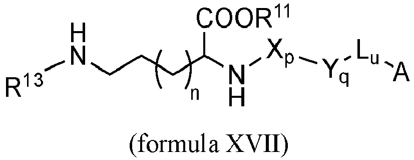 is obtained. The moiety A corresponds to the moiety A of general formula I with the provision that reactive side chains can be protected by protecting groups. For example, -COOH may be protected as -COOtBu and PMB-protecting groups may be on the thiol-groups of A.

In a sixth step, a Dde cleavage of the compound of general formula XVII is performed whereby a reaction product of general formula XVIII 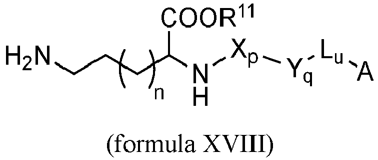 is obtained. In a seventh step, an amide coupling between the reaction product of general formula XVIII and a compound of general formula IV 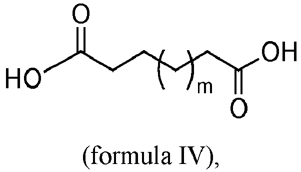 is performed, whereby a reaction product of general formula XIX 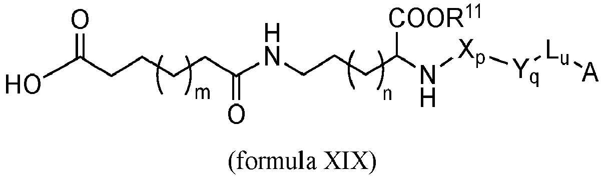 is obtained. In an eight step, an amide coupling between the reaction product of general formula XIX and a compound of general formula VI 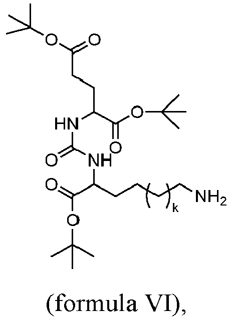 is performed, whereby a reaction product of general formula XII 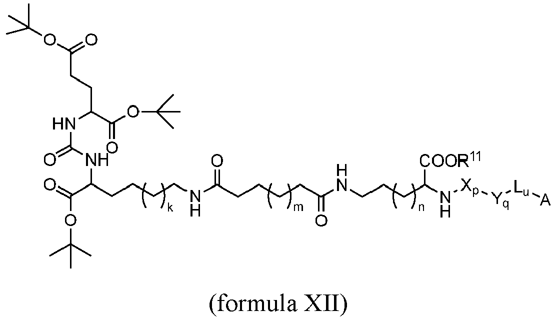 is obtained. In a next step, the reaction product of general formula XII is cleaved from the 2-CTC resin and deprotected by removing all protective groups whereby the compound of general formula I is obtained. In formulae XII to XIX, the indices k, m, n, p, q, u, v, x, y and z have the meanings given in the context of general formula I.

The synthesis of the compounds of general formula I is described in further detail in the example section.

The N₂S₂ chelator incorporated in the compounds of general formula I can be used to label these PSMA ligands with different metals by means of complex formation. The complexes are of use as diagnostics and therapeutics of diseases in which PSMA is involved, such as prostate cancer. The complexes are of use as diagnostics and therapeutics of certain diseases where PSMA is upregulated. One example of a disease where PSMA is upregulated is prostate cancer.

Surprisingly, it has been found in a LNCaP xenograft model that in particular compound 5 exhibits faster pharmacokinetic properties and a more favorable organ distribution (high tumor uptake, low to moderate uptake in non-target organs such as kidneys) than those known from the prior art. High tumor-to-muscle and tumor-to-kidney ratios are reached as early as 1 hour after injection which allows for imaging at early timepoints. A further increase of the tumor-to-muscle and the tumor-to-kidney ratio over time was observed. These findings suggest a better visualization of the tumor compared to the compounds known from the prior art.

To demonstrate the influence of the moieties X and Y of the inventive compounds, the inventors have synthesized a comparative compound of the general formula BM-Lᵤ-A, wherein BM, L, A and u have the meanings given in context with general formula I. The compound of the general formula BM-Lᵤ-A is a compound of the general formula I where p is 0 and q is 0. An example of a comparative compound BM-Lᵤ-A is comparative compound 3 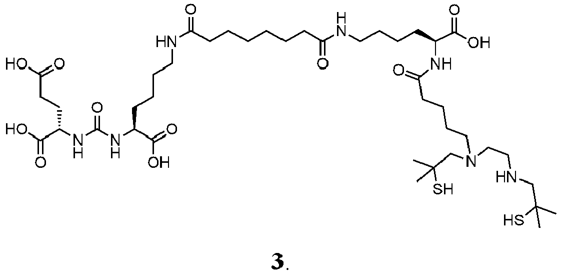 A comparison of compound 5 with compound 3 revealed that the advantageous properties of the compounds of general formula I could result from the presence of the amino acids X and Y in the compound of the general formula I. Without being bound by an explanation, the inventors believe that the faster pharmacokinetic properties and a more favorable organ distribution (high tumor uptake, low to moderate uptake in non-target organs such as kidneys) of compound 5 in comparison with comparative compound 3 could be explained by the presence of the moieties X and Y in the compounds of general formula I.

Compared to the United States Food and Drug Administration (FDA) approved, well studied and widely used prostate cancer imaging agent ⁶⁸Ga-PSMA-11, which is a PET tracer, the complexes of ^{99m}Tc and compounds of general formula I were surprisingly found to have similar properties in the in vivo and in vitro experiments described herein. The compound of the present invention displayed similar affinity for PSMA, higher internalization in LNCaP tumor cells and comparable tumor uptake values in a LNCaP xenograft model when compared 1 hour after injection of the respective compound. Since technetium and rhenium represent a theranostic pair, the inventors expect similar properties for complexes of ¹⁸⁶Re and compounds of general formula I and complexes of ¹⁸⁸Re and compounds of general formula I.

Thus, the complexes according to the invention can be used as a medicament, in particular a medicament for diseases in which PSMA is involved. Also, a pharmaceutically acceptable salt of a complex according to the invention can be used as a medicament, in particular a medicament for diseases in which PSMA is involved. In one embodiment, the complexes according to the invention can be used as a medicament in the diagnosis and therapy of diseases in which PSMA is involved. Also, a pharmaceutically acceptable salt of a complex according to the invention can be used as a medicament in the diagnosis and therapy of diseases in which PSMA is involved. Diseases in which PSMA is involved for example are diseases where PSMA is upregulated. One example of a disease where PSMA is upregulated is prostate cancer.

Thus, according to the invention provided is the use of a complex according to the invention as a medicament. Further provided is the use of a complex according to the invention as a medicament in the diagnostics and therapy of diseases in which PSMA is involved. The medicament can be a radiopharmaceutical. Preferably, the medicament is a radiopharmaceutical for nuclear medical imaging. For example, the medicament is a radiopharmaceutical for nuclear medical imaging by means of single-photon emission computed tomography (SPECT) or of positron emission tomography (PET). Also preferably, the medicament is a radiopharmaceutical for the radioligand therapy. In a preferred embodiment, an inventive complex comprising a metal selected from the group consisting of ^{99m}Tc, ¹⁸⁶Re, ¹⁸⁸Re and ⁶⁷Cu is used as a radiopharmaceutical for nuclear medical imaging by means of single-photon emission computed tomography (SPECT). In another preferred embodiment, an inventive complex comprising ^{94m}Tc or ⁶⁴Cu is used as a radiopharmaceutical for nuclear medical imaging by means of positron emission tomography (PET). In still another preferred embodiment, an inventive complex comprising a metal selected from the group consisting of ¹⁸⁶Re, ¹⁸⁸Re, ⁶⁴Cu and ⁶⁷Cu is used as a radiopharmaceutical for the radioligand therapy. Instead of a medicament containing a compound of general formula I according to the invention use can be made of a pharmaceutically acceptable salt of said compound. Due to its long half life of 211,000 years, an inventive complex comprising ⁹⁹Tc may be used for structural elucidation of the geometry of the inventive complexes.

According to the invention, there is further provided a method for preparing a complex comprising a compound of general formula I as ligand and a metal. The method comprises the step of contacting the compound of general formula I with the metal. In a preferred embodiment, the compound of general formula I is contacted with the metal at a reaction temperature in the range of from 20 to 100°C. Preferably, the compound of general formula I according to the invention is contacted with the metal at room temperature. Preferably, the compound of general formula I is contacted with the metal at ambient pressure. It may be provided that the molar ratio of the compound of general formula I to a non-radioactive metal ion in the solution is 1 : 1. Preferably, the method according to the invention is carried out with a protic solvent, for example water. The pH value may be chosen in dependence on the used metal.

In the following, the invention is explained in more detail with the help of examples not intended to limit the invention with respect to the drawings. Here
- Fig. 1: shows functional imaging of subcutaneous LNCaP tumor xenografts in mice using [^{99m}Tc]TcO-5 [= [^{99m}Tc]TcO-ABX474] compared to reference compounds;
- Fig. 2: shows diagrams illustrating tumor-to-background kinetics of [^{99m}Tc]TcO-5 [= [^{99m}Tc]TcO-ABX474] compared to reference compounds in LNCaP tumor bearing mice; and
- Fig. 3: shows a diagram illustrating the tumor-uptake of [^{99m}Tc]-labeled radioligands in LNCaP tumor bearing mice.

### Examples

### General method for the synthesis of compounds 1, 2, 4, 5, and 6

Compounds **1, 2, 4, 5,** and **6** were synthesized via solid phase peptide synthesis (SPPS) on 2-chlorotrityl resin.

Analysis of the synthesized molecules was performed using reversed-phase high performance liquid chromatography (RP-HPLC; Ascentis Express C18, 150x4.6 mm; Supelco, Germany) with a linear A-B gradient (5% B to 100% B in 10 min) at a flow rate of 1.5 mL/min (analysis). Purification was performed using reversed-phase high performance liquid chromatography (RP-HPLC; Gemini-NX C18, 250x50 mm; Phenomenex, Germany) with a linear A-B gradient at a flow rate of 100 mL/min. Solvent A consisted of 0.1 % aqueous TFA and solvent B was 0.1 % TFA in ACN.

The HPLC system (Dionex Ultimate 3000; Thermo-Fisher, Germany) was equipped with a UV detector. UV absorbance was measured at 200, 210 and 230 nm. Mass spectrometry was performed with a LC-MS System (Dionex 3000, Thermo-Fisher, Germany).

### Examples 1 to 4

### Synthesis of Intermediates

To prepare compounds **1** to **6** the following intermediates have been prepared.

### Example 1

### Synthesis of intermediate 101: Glu-CO-Im ((di-tert-butyl (1H-imidazole-1-carbonyl)-L-glutamate))

H-Glu(OtBu)-OtBu (29.59 g, 1 eq. 100 mmol) was dissolved in 400 ml DCM. Triethylamine (25.3 g, 2.5 eq., 250 mmol) was added slowly. Carbonyldiimidazole (17.84 g, 1.1 eq., 110 mmol) was added in small portions. Reaction was left agitating for 4 hours. The solution was washed with water, NaHCO₃ and brine. The organic phase was dried over Na₂SO₄ and evaporated in vacuo to give the target compound **101** as an oil.

### Example 2

### Synthesis of Intermediate 103: N'-[2-(4-Methoxy-benzylsulfanyl)-ethyl]-N'-{2-[4-methoxy-benzylsulfanyl)-ethylamino]-ethyl}-propan-1,3-diamine

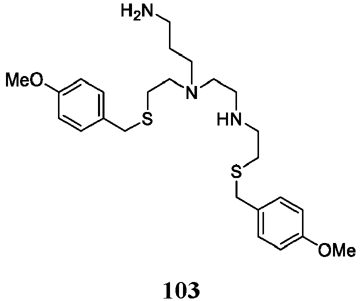

The synthesis of intermediate **103** was performed analogously to WO 2012/022812 A1.

### Step 1: 2-(4-Methoxy-benzylsulfany)-ethylamine (compound 104)

Sodium (4.5 g, 196 mmol) was added to vigorously stirred methanol (150 ml, dry). When the sodium is completely dissolved, 2-aminoethanethiol hydrochloride (10.8 g 95.0 mmol) was added. After that, p-methoxybenzylchloride (14.9 g, 95.5 mmol) was added via a dropping funnel. The mixture was heated under reflux at 70°C for 30 minutes. Subsequently the mixture was cooled to room temperature. The solid was removed by filtration and the filter cake washed with methanol (3 times with 25 ml). The organic extracts were combined and volatiles removed under reduced pressure. This residue was re-dissolved in DCM (75 ml), extracted with water (75 ml × 3), dried (MgS0₄), filtered and solvent removed to leave compound **104** a colorless oil. Yield 18.5 g (99 %).

### Step 2: N-[(4-Methoxy-benzylsulfanyl)-ethyl]-2-chloroacetamide (compound 105)

Chloroacetyl chloride (4.24 ml, 53.2 mmol) in dry DCM (50 ml) was added dropwise over 90 minutes with stirring to an ice bath cooled (0°C) solution of 2-(4-methoxy-benzylsulfanyl)-ethylamine **104** (9.4 g, 47.5 mmol) and triethylamine (8.0 ml) in dry DCM (200 ml). After addition the cooling bath was removed and stirring continued for 60 minutes. The solution was extracted with water (2 × 250 ml), dried (MgSO₄), filtered and solvent evaporated under reduced pressure to leave compound **105** as a colored solid. Yield 12.93 g (99 %).

### Step 3: Methyl 3-[(4-methoxy-benzylsufanyl)-ethylamino]-propanoate (compound 106)

Methyl acrylate (4.46 ml 49.2 mmol) in methanol (10 ml) was added to a stirred solution of 2-(4-methoxy-benzylsulfanyl)-ethylamine **104** (8.85 g, 48.3 mmol) in methanol (50 ml). The colorless solution was allowed to stir at r.t. for 6 hours. Volatiles were removed by rotary evaporation to leave compound **106** as colorless viscous oil. Yield 1.35 g (97 %).

### Step 4: 3-[2-(4-Methoxybenzylsulfanyl)-ethylamino]-propanamide (compound 107)

Methyl 3-[(4-Methoxy-benzylsufanyl)-ethylamino] propanoate **106** (10.65 g 37.6 mmol), methanol (120 ml) and ammonia solution (200 ml) were stirred at r.t. for 24 hours. Volatiles were removed under reduced pressure to give compound **107** as an almost white solid. Yield 9.98 g (99 %).

### Step 5: 3-([2-(4-Methoxy-benzylsulfany)-ethyl]-{[2-(4-methoxy-benzylsulfanyl)-ethylcarbamoyl]-methyl}-amino)-propionamide(compound 108)

3-[2-(4-Methoxybenzylsulfanyl)-ethylamino] propenamide **106** (10.33 g, 38.5 mmol), N-[(4-Methoxy-benzylsulfanyl)-ethyl]-2-chloroacetamide **105** (10.54 g, 38.5 mmol), triethylamine (6.5 ml) and acetonitrile (80 ml) were heated at 70°C overnight. Subsequently the mixture was cooled to room temperature. The solvent was removed under reduced pressure to give a brown residue which was purified over silica eluting with DCM/Methanol 20:1 to yield compound **108** as a colorless oil (yield 8.3 g, 43 %).

### Step 6: N'-[2-(4-Methoxy-benzylsulfanyl)-ethyl]-N'-{2-[4-methoxy-benzylsulfanyl)-ethylamino]-ethyl}-propan-1,3-diamine (compound 109)

1.0M borane in THF (102 ml, 102 mmol) was added via a syringe under argon atmosphere to 3-([2-(4-Methoxy-benzylsulfanyl)-ethyl]-{[2-(4-methoxybenzyl-sulfanyl)-ethylcarbamoyl]-methyl}-amino)-propionamide **108** (3.8 g, 7.5 mmol). The resulting colorless solution was heated under reflux at 70°C overnight. After cooling to r.t. water (40 ml) was added dropwise. The solvent was removed under reduced pressure to leave a waxy solid which was diluted with HCl (0.5 N, 400 ml). The mixture was heated under reflux at 100°C for 3 hours. After cooling to r.t. sodium hydroxide was added until a pH 10-11 was obtained. This mixture was extracted with DCM (4 × 200 ml) and the organic fractions were combined, dried (MgSO₄) and filtered. The solvent was evaporated to leave a waxy solid, which was purified over silica eluting with DCM/Methanol/NH₄OH = 9:1:0.1 to yield compound **109** as a colorless oil. Yield 1.29 mg (36 %)

### Example 3

### Synthesis of Intermediate 4: N-Boc-N'-(5-carboethoxypentyl-N,N'-bis-(2-(4-methoxy-benzylthio)-2-methylpropyl)-ethylenediamine (compound 110)

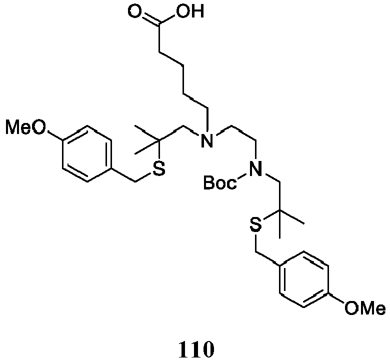

The synthesis of intermediate **110** was performed analogously to US 5 776 428 A.

### Step 1: 2,2'-Dithiobis(2-methylpropanal) (compound 111)

To 2-Methylpropanal (28.8 g, 0.4 mol) in carbon tetrachloride (30 ml) was added sulfur monochloride (27 g, 0.2mol). The reaction mixture was stirred at 50 °C for 16 hours. After cooling to room temperature, the volatiles were evaporated in vacuo and residue was purified by distillation in vacuo (bp at 0.5 Torr: 98 - 102 °C) to yield 22.7 g (55%) of 2,2'-dithiobis(2-methylpropanal) **111.**

### Step 2: 3,3,10,10-tetramethyl-6,7-dihydro-1,2,5,8-dithiadiazecine (compound 112)

To a stirred solution of 2,2'-dithiobis(2-methylpropanal) **111** (5 g) in chloroform (20 mL) were added 1.8 g of ethylenediamine. The reaction mixture was stirred at room temperature for 2 hours. After the removal of the solvent the residue was triturate with water until crystals began to form. The white crystals were collected by filtration and washed with ethanol to yield 4.8 g (86%) of 3,3,10,10-tetramethyl-6,7-dihydro-1,2,5,8-dithiadiazecine **112.**

### Step 3: N,N'-Bis(2-mercapto-2-methylpropyl)ethylene diamine (compound 113)

To 4.1 g (18 mmol) of 3,3,10,10-tetramethyl-6,7-dihydro-1,2,5,8-dithiadiazecine **112,** dissolved in 70 ml of dry THF were added with stirring (argon atmosphere) 1.35 g of LiAlH₄ (0.36 mmol). The reaction mixture was refluxed for 4 hours and subsequently hydrolyzed by the cautious addition of saturated NaK-tartrate solution (20 ml), and then diethyl ether (100 ml) were added. The sludge was separated by decanting or filtration (Celite) and washed well with ether. The solvent was removed in vacuo to yield 0.8 g (20%) of the compound **113.**

### Step 4: N,N'-bis-(2-(4-methoxybenzylthio)-2-methylpropyl)-ethylenediamine (compound 114)

A solution of N,N'-bis(2-mercapto-2-methylpropyl)ethylene diamine **113** (1.1g, 4.65 mmol) in methanol (50 mL) was cooled in ice/water bath and then saturated with gaseous ammonia over 30 min. To this was added 4-methoxybenzyl chloride (1.9 g,12.3 mmol). The reaction was allowed to warm to room temperature overnight with stirring under argon. Methanol was evaporated in vacuo and the residue was then partitioned between diethyl ether (50 mL) and 0.5M KOH (40 mL). The aqueous layer was further extracted with diethyl ether (2x25 mL). The combined organic layers were washed with NaCl solution and concentrated in vacuo to give a clear colorless oil. The oil was dissolved in diethyl ether (200 mL) and then acidified with 4.0M HCl in dioxane. The white precipitate was collected by filtration and washed with diethyl ether. The HCl salts were partitioned between 1M KOH (30 mL) and ethyl acetate (30 mL). The aqueous layer was extracted with ethyl acetate (2 × 30 mL) and the combined organic layers were washed with NaCl solution, dried over Na₂SO₄ and concentrated to give the pure compound **114** (free base) as a light-yellow oil (0.99g. 45 % yield).

### Step 5: N-(5-carboethoxypentyl- N,N'-bis-(2-(4-methoxybenzylthio)-2-methylpropyl- ethylenediamine (compound 115)

To N,N'-Bis-(2-(4-methoxybenzylthio)-2-methylpropyl)-ethylenediamine **114** (921 mg, 1.93 mmol) in acetonitrile (15 mL) was added K₂CO₃ (270 mg) followed by ethyl 5-bromovalerate (807 mg). The reaction was stirred at reflux overnight and subsequently concentrated in vacuo. The residue was partitioned between ethyl acetate (50 mL) and 0.5M KOH (50 mL). The aqueous layer was extracted with ethyl acetate (2×50 mL) and the combined organic layers were washed with brine (50 mL), dried over Na₂SO₄ and concentrated to give a yellow oil, which was purified over silica eluting with DCM/Methanol = 20:1 to yield the desired compound **115** as a yellowish oil. Yield 635 mg (54 %)

### Step 6: N-Boc-N-(5-carboethoxypentyl-N,N-bis-(2-(4-methoxybenzylthio)-2-methylpropyl)-ethylenediamine (compound 116)

To N-(5-carboethoxypentyl)-N,N'-bis-(2-(4-methoxybenzylthio)-2-methylpropyl) ethylenediamine **115** (635 mg 1.05 mmol) in THF (40 mL) was added water (30 mL) and 1M KOH (2.5 mL, 2.5 mmol). The homogeneous solution was refluxed overnight. The solution was then cooled to room temperature and the THF was removed in vacuo. The residue was diluted with 50 mL water and the pH was adjusted to 2-3 by addition of 1M HCl. The solution was extracted with ethyl acetate (3x50 mL). The combined organic layers were washed with brine (50 mL), dried over Na₂SO₄ and concentrated in vacuo to give 575 mg of a crude intermediate.

The crude intermediate was dissolved in ACN (50 mL) and Boc₂O (326 mg) followed by triethylamine (0.280 mL) were added. The homogenous solution was stirred at room temperature overnight under argon. The solution was then concentrated in vacuo and subsequently partitioned between ethyl acetate (25 mL) and 1M KH₂PO₄ (25 mL). The organic layer was washed with 5% citric acid (2x25 mL) and brine (25 mL), dried over Na₂SO₄ and concentrated to give a yellow oil (890 mg). Purification by column chromatography over silica eluting with DCM/Methanol = 20:1 yielded compound **116** as a yellowish oil. Yield 455 mg (64%).

### Example 4

### Synthesis of compound 5 [ABX 474]

The synthesis of the inventive compound **5** 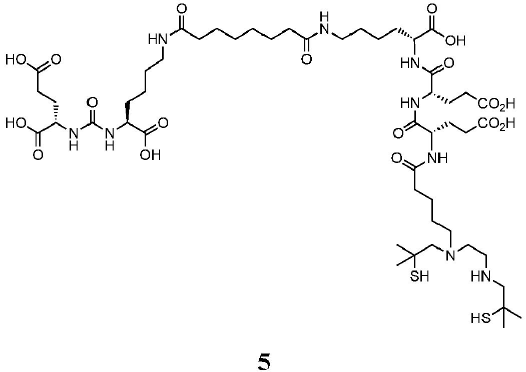 is shown in Scheme EX-1. 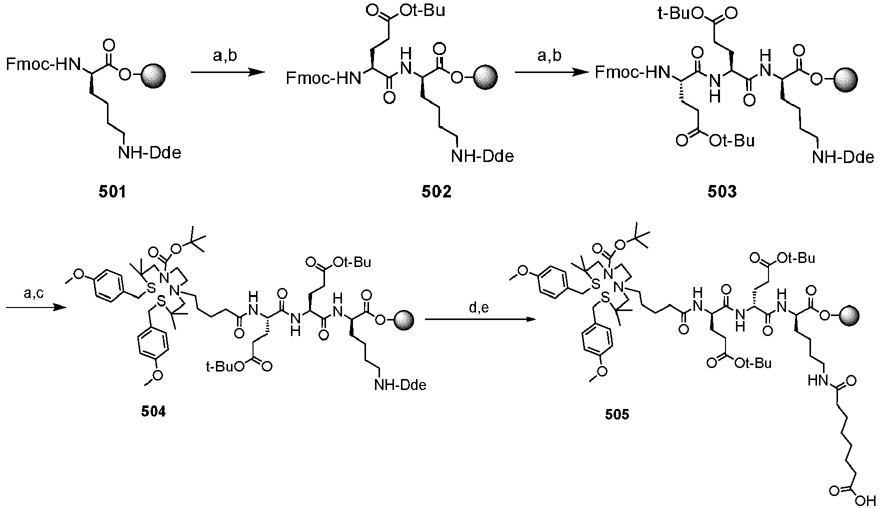 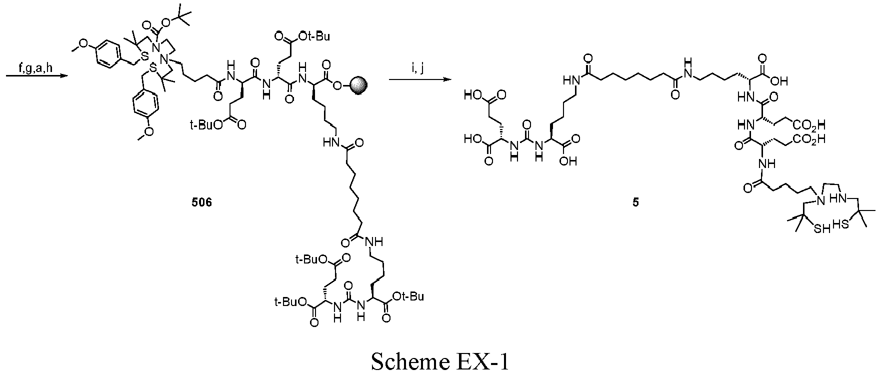 In Scheme EX-1, letter a) indicates the use of 20% Piperidine in DMF; letter b) the use of Fmoc-Glu(OtBu)-OH, HATU, HOAt, DIPEA, and DMF; letter c) the use of Intermediate **110,** PyBOP, DIPEA, and DMF; letter d) the use 2% N₂H₂.H₂O in DMF; letter e) the use of Sub-NHS, DIPEA, and DMF; letter f) the use of TSTU, DIPEA and DMF; letter g) the use of Fmoc-Lys-OtBu, DIPEA, and DMF; letter h) the use of Glu-CO-Im, NMM, and DMF; letter i) the use of TFA:TIS:H₂O:EDT (92.5:2.5:2.5:2.5); and letter j) the use of 10% TFMSA.

For synthesizing of compound **501,** Fmoc-D-Lys(Dde)-OH was loaded to 2-CTC resin (in Schema EX-1, the resin is represent by a circle) and Fmoc was deprotected with 20% piperidine in DMF. Then, for synthesizing of compounds **502** and **503,** Fmoc-Glu(OtBu)-OH (2 eq.) was activated with HATU (2 eq.), HOAt (2 eq.) and DIPEA (5,6 eq.) in DMF and added to the resin. The reaction mixture was agitated for 2 h at room temperature (r.t.). Fmoc-deprotection was performed with 20% piperidine in DMF. For synthesizing compound **504,** intermediate **110** was conjugated to the peptide sequence using PyBOP (2 eq.) and DIPEA (2 eq.) in DMF for 2 h at r.t. Dde-deprotection was conducted using 2% hydrazine monohydrate in DMF. Afterwards, for synthesizing of compound **505,** suberic acid-mono-NHS ester (2 eq.) and DIPEA (2eq.) were dissolved in DMF (1 mL) and allowed to react with the resin bound peptide for 2 h at r.t. Then, for synthesizing of compound **506,** the free carboxylic acid was treated with TSTU (2 eq.) and DIPEA (2 eq.) in DMF (10 mL/g resin) at r.t. for 1h. After the formation of NHS ester, the resin bound peptide was treated with Fmoc-Lys-OtBu (2 eq.) and DIPEA (2 eq.) in DMF (2 mL) for 2 h at r.t. Fmoc was cleaved using 20% piperidine in DMF and then the free amine was treated with Glu-CO-Im (Intermediate **101,** 3 eq.), and NMM (3 eq.) in DMF for 2 h at r.t. After final coupling, the resin was washed with DMF (3 × 5 mL), DCM (3 × 5 mL), IPA (3 × 5 mL), and Et₂O (3 × 5 mL). For synthesizing of compound **5,** cleavage from resin and final deprotection was performed by treatment with TFA/TIS/EDT/water (v/v/v/v; 92.5/2.5/2.5/2.5) at 0 °C and subsequent dropwise addition of 10% TFMSA.

The crude peptide was precipitated from ice-cold diethyl ether and purified via RP-HPLC. After RP-HPLC purification, compound **4** [ABX 474] (TFA salt) was obtained as a white to off white solid (isolated yield 38%). Calculated monoisotopic mass (C₅₁H₈₉N₉O₁₈S₂): 1179.58; found: m/z = 1180.5[M+H]⁺, 590.79 [M+2H]²⁺.

### Example 5

### Synthesis of compound 6 [ABX 490]

The synthesis of the inventive compound **6** 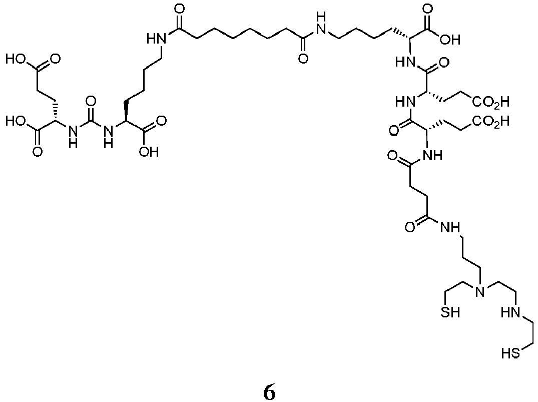 is shown in Scheme EX-2. 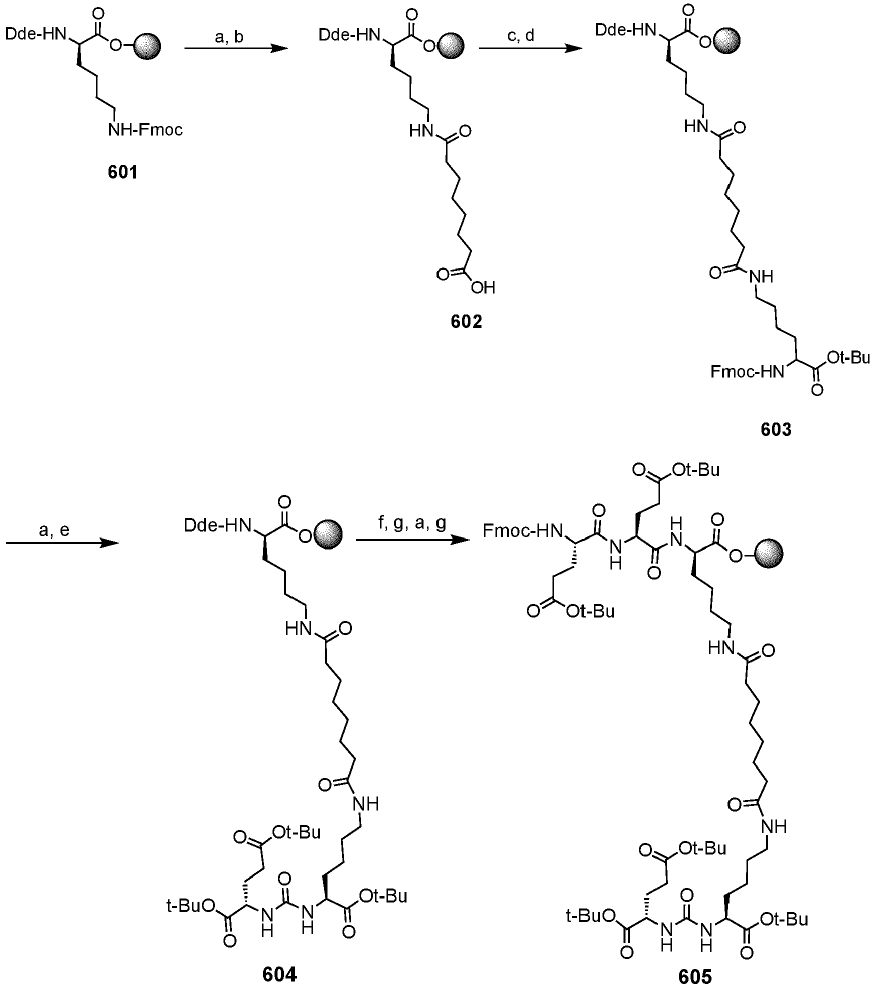 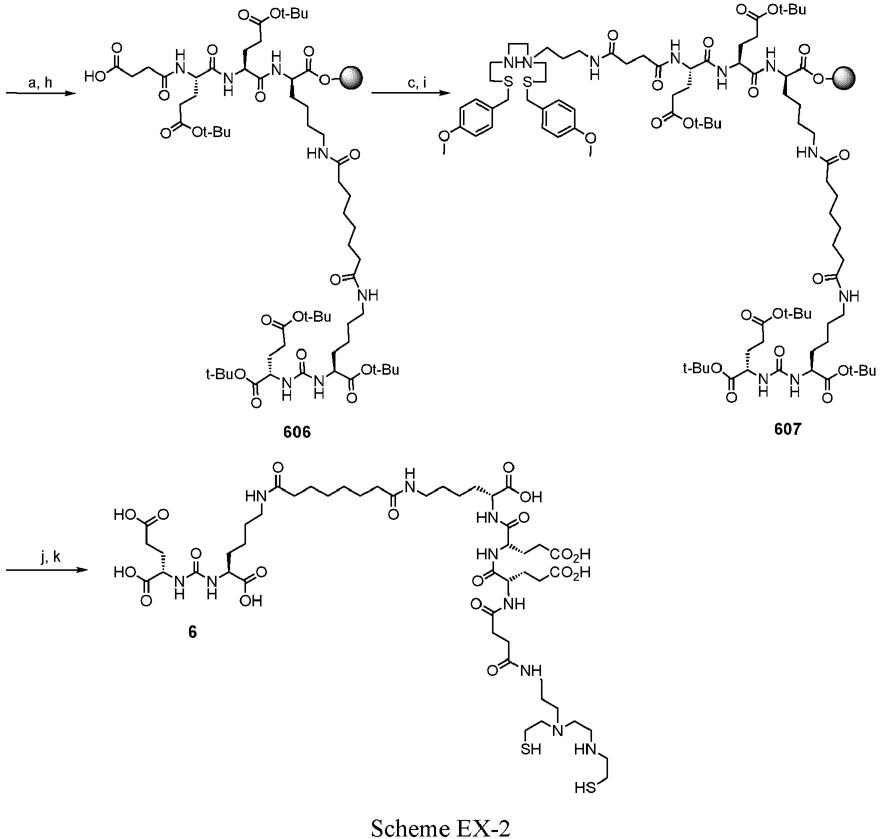 In Scheme EX-2, letter a) indicates the use of 20% Piperidine in DMF; letter b) the use of Sub-NHS, DIPEA, and DMF; letter c) the use of TSTU, DIPEA and DMF and a reaction time of 1 h; letter d) the use of Fmoc-Lys-OtBu, DIPEA, and DMF; letter e) the use of Glu-CO-Im, NMM, and DMF; letter f) the use of 2% N₂H₂.H₂O in DMF; letter g) the use of Fmoc-Glu(OtBu)-OH, PyBop, HOBt, DIPEA, and DMF; letter h) the use of succinic anhydride, DIPEA, and DMF; letter i) the use of intermediate **103,** DIPEA, and DMF; letter j) the use of TFA:TIS:H₂O:EDT (92.5:2.5:2.5:2.5); and letter k) the use of 10% TFMSA.

For synthesizing compound **601,** Dde-D-Lys(Fmoc)-OH was loaded to the 2-CTC resin (in Schema EX-2, the resin is represent by a circle) and Fmoc was deprotected with 20% piperidine in DMF. Afterwards, for synthesizing compound **602,** suberic acid-mono-NHS ester (2 eq.) and DIPEA (2eq.) were dissolved in DMF (1 mL) and allowed to react with resin bound peptide for 2 h at r.t. Then, for synthesizing compound **603,** the free carboxylic acid was treated with TSTU (2 eq.) and DIPEA (2 eq.) dissolved in DMF (2 mL) and reacted for 1 h at r.t. After the formation of NHS ester, resin bound peptide was treated with Fmoc-Lys-OtBu (2 eq.) and DIPEA (2 eq.) in DMF (1 mL) for 2 h at r.t. For synthesizing compound **604,** Fmoc was cleaved using 20% piperidine in DMF and then the free amine was treated with Glu-CO-Im **101** (3 eq.), and NMM (3 eq.) in DMF for 2 h at r.t. Dde-deprotection was conducted using 2% hydrazine monohydrate in DMF. Afterwards, for synthesizing compound **605,** Fmoc-Glu(OtBu)-OH (2 eq.) was activated with PyBOP (2 eq.), HOBt (2 eq.) and DIPEA (2 eq.) in DMF and added to the resin. The reaction mixture was agitated for 2 h at r.t. For synthesizing compound **606,** Fmoc was deprotected with 20% piperidine in DMF. Succinic anhydride (2 eq.) and DIPEA (2 eq.) dissolved in DMF (2 mL) was added to resin bound peptide and agitated for 2 h at r.t. Then the free carboxylic acid was activated with TSTU (2 eq.) and DIPEA (2 eq.) dissolved in DMF (2 mL) and reacted for 1h at r.t. After the formation of NHS ester, resin bound peptide was treated with intermediate **103** (1 eq.) and DIPEA (2 eq.) in DMF (2 mL) for 2 h to synthesize compound **607.** After completion of the reaction, resin was washed with DMF (3 × 5 mL), DCM (3 × 5 mL), IPA (3 × 5 mL), and Et₂O (3 × 5 mL). For synthesizing compound **6,** cleavage from resin and final deprotection was performed by treatment with TFA/TIS/EDT/water (v/v/v/v; 92.5/2.5/2.5/2.5) at 0 °C and subsequent dropwise addition of 10% TFMSA.

The crude peptide was precipitated from ice-cold diethyl ether and purified *via* preparative RP-HPLC. After RP-HPLC purification, compound 6 [ABX 490] (TFA salt) was obtained as a white to off white solid (yield: 12%). Calculated monoisotopic mass (C₄₉H₈₄N₁₀O₁₉S₂): 1180.54; found: m/z = 1181,38 [M+H]⁺, 591.27 [M+2H]²⁺.

### Example 6

### Synthesis of compound 1 [ABX 408]

Inventive compound **1** 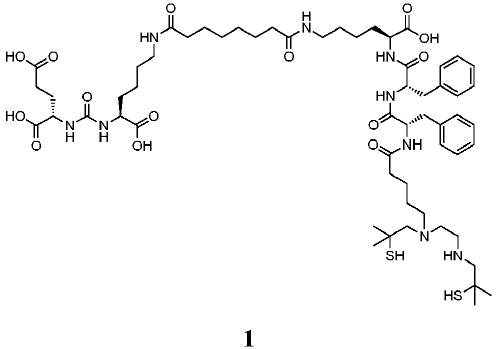 was synthesized by means of the procedure described in Example 4 apart from the use of Fmoc-L-Lys(Dde)-OH instead of Fmoc-D-Lys(Dde)-OH and Fmoc-Phe-OH instead of Fmoc-Glu(OtBu)-OH. The crude peptide was purified *via* preparative RP-HPLC whereby compound 4 was obtained. Calculated monoisotopic mass (C₅₉H₉₃N₉O₁₄S₂): 1215.63; found: m/z = 1214.73 [M-H]⁻.

### Example 7

### Synthesis of compound 2 [ABX 451]

Inventive compound **2** 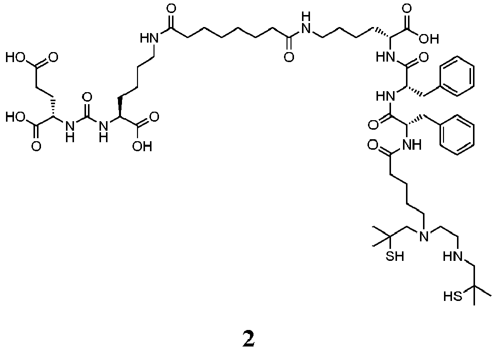 was synthesized by means of the procedure described in Example 4 apart from the use of Fmoc-Phe-OH instead of Fmoc-Glu(OtBu)-OH. The crude peptide was purified *via* preparative RP-HPLC. Calculated monoisotopic mass (C₅₉H₉₃N₉O₁₄S₂): 1215.63; found: m/z = 1216.70 [M+H]⁺.

### Comparative Example 1

### Synthesis of compound 3 [ABX 455]

Comparative compound **3** 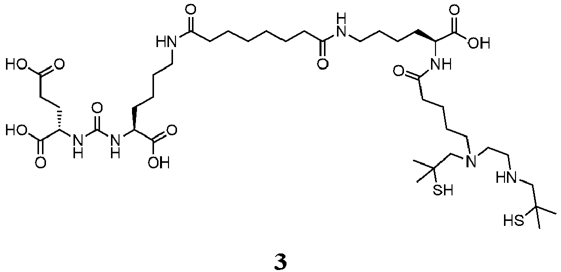 was synthesized by means of the procedure described in Example 4 apart from the omission of Fmoc-Glu(OtBu)-OH. The crude peptide was purified *via* preparative RP-HPLC. Calculated monoisotopic mass (C₄₁H₇₅N₇O₁₂S₂): 921.49; found: m/z = 922.45 [M+H]⁺.

### Example 8

### Synthesis of compound 4 [ABX 456]

Inventive compound **4** 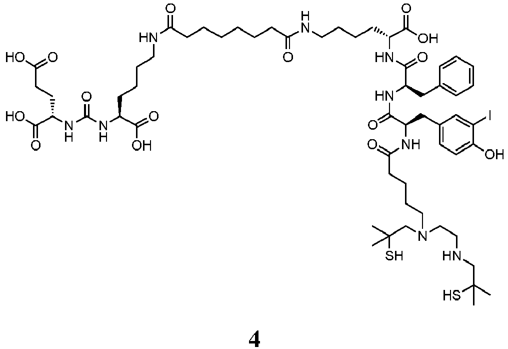 was synthesized by means of the procedure described in Example 4 apart from the use of Fmoc-D-Phe-OH and Fmoc-D-Tyr(3I)-OH instead of Fmoc-Glu(OtBu)-OH. The crude peptide was purified *via* preparative RP-HPLC. Calculated monoisotopic mass (C₅₉H₉₂IN₉O₁₅S₂): 1357.52; found: m/z = 1356.70 [M-H]⁻.

### Example 9

### Synthesis of ^{nat}Re-Complexes

The compounds **1, 3** and **5** were used to synthesize complexes with naturally abundant Re isotopes (^{nat}Re). These synthesized complexes can be used as PSMA ligands.

0.06 mmol of the corresponding compound **1, 3** or **5** were dissolved in 2 ml of methanol. Subsequently 1 ml of a 1 NNaOAc solution and 0.06 mmol oxotrichloro[(dimethylsulfide)-triphenylphosphine oxide]-rhenium(V) were added. The reaction mixture was stirred at room temperature overnight. The crude product was precipitated with ice-cold diethyl ether and purified *via* preparative RP-HPLC. Table Ex-1 shows the results of the LC-MS analysis of the synthesized rhenium complexes.

**Table EX-1**

| | calculated monoisotopic mass | found |
|---|---|---|
| ^{nat}ReO-**1** [= ^{nat}ReO-ABX408] | 1415.55 | m/z = 1416.53 [M+H]⁺ |
| ^{nat}ReO-**3** [= ^{nat}ReO-ABX455] | 1121.42 | m/z = 1122.58 [M+H]⁺ |
| ^{nat}ReO-**5** [= ^{nat}ReO-ABX474] | 1379.50 | m/z = 690.92 [M+2H]²⁺, 1380.08 [M+H]⁺ |

The ^{nat}-Re-complexes of the compounds **1, 3** and **5** can be used as standards for the radiosynthesis of ¹⁸⁶Re and ¹⁸⁸Re-labeled PSMA-ligands. Analogously, the ^{nat}-Re-complexes of the compounds 2, 4 and 6 can be used as standards for the radiosynthesis of ¹⁸⁶Re and ¹⁸⁸Re-labeled PSMA-ligands.

### Example 10

### Radiolabeling of compounds 1 to 6 with ^{99m}Tc

The compounds 1 to 6 were used to synthesize complexes with ^{99m}Tc. These synthesized complexes can be used as PSMA radioligands.

The corresponding compound **1, 2, 3, 4, 5** or **6** (50 µg), mannitol (1 mg), and calcium heptagluconate (10 µg) were stirred in 1-1.5 mL sodium pertechnetate / saline solution (from commercially available ⁹⁹Mo/^{99m}Tc generator, 0.2-4 GBq) at r.t. for 10 min. After addition of stannous chloride (1 µg in 100 µL of 0.01 N HCl; solution saturated with helium) the solution was kept at r.t. for 20 min and finally at 80°C for another 20 min. After cooling down, the product was ready for further use. The product is a complex of ^{99m}Tc and the corresponding compound **1, 2, 3, 4, 5** or **6.** These complexes were provided as a solution of the respective complexes.

Quality control of the product was performed by radio-reversed-phase-high-performance liquid chromatography (radio-RP-HPLC) using a Poroshell 120 EC-C18 column (3.5 µm, 100 × 3 mm; Agilent Technologies Deutschland, Waldbronn, Germany) and a solvent system consisting of water (0.1 % TFA) and ACN (0.1 % TFA). Analyses were performed using two different gradient elution methods: a) 0-0.5 min 0 %, 0.5-3 min 0-100 %, 3-5 min 100 %, 5-8 min 0 % ACN (0.1 % TFA); flow rate: 0-0.5 min 0.4 mL/min, 0.5-8 min 0.7 mL/min and b) 0-1.5 min 0 %, 1.5-10 min 0-100 %, 10-12 min 100 %, 12-15 min 0 % ACN (0.1 % TFA); flow rate: 0.7 mL/min, as well as isocratic elution methods using a particular percentage of ACN (0.1% TFA) appropriate for the respective radioligand. In addition, for determination of possible ^{99m}Tc/⁹⁹Tc-colloid formation, radio-thin layer chromatography (radio-TLC) was perfomed on silica gel TLC sheets (POLYGRAM SIL G UV254, Macherey-Nagel, Düren, Germany) using methanol /ammoniumacetate (2 M) 1:1. Table 2 shows the radiochemical purity and the retention time t_{R} of the obtained complexes.

**Table EX-2**

| Radioligand | Radiochemical purity^{∗} | t_{R}^{∗} |
|---|---|---|
| [^{99m}Tc]TcO-**1** [=[^{99m}Tc]TcO-ABX408] | 92.2 % | 3.64 min |
| [^{99m}Tc]TcO-**2** [=[^{99m}Tc]TcO-ABX451] | 93.0% | 3.45 min |
| [^{99m}Tc]TcO-**3** [=[^{99m}Tc]TcO-ABX455] | 93.6% | 3.34 min |
| [^{99m}Tc]TcO-**4** [=[^{99m}Tc]TcO-456] | 94.5 % | 3.46 min |
| [^{99m}Tc]TcO-**5** [=[^{99m}Tc]TcO-ABX474] | 96.7 % | 4.16 min |
| [^{99m}Tc]TcO-**6** [=[^{99m}Tc]TcO-ABX4901 | 94.3 % | 3.65 min |

| | | |
|---|---|---|
| ^{∗} determined by radio-RP-HPLC, method a) | | |

### Example 11

### Determination of logD values

LogD values were determined for different media by the shake-flask method (Andrés, A.; Roses, M.; Ràfols, C.; Bosch, E.; Espinosa, S.; Segarra, V.; Huerta, J. M. Setup and validation of shake-flask procedures for the determination of partition coefficients (logD) from low drug amounts. Eur. J. Pharm. Sci. 2015, 76, 181-191.). In preparation, octanol was saturated with each of the used buffer solutions and vice versa. To 3 mL of octanol, together with 3 mL of buffer solution 200-300 µL of the ^{99m}Tc-labeled radioligand (15-25 MBq, samples in triplicate) were added and shaken vigorously for 30 min. After centrifugation at 9,000 rpm for 15 min, samples were taken from both separated phases and measured by gamma-counter. LogD values were calculated as the ratio of radioactivity determined in octanol and aqueous phase. Table EX-3 shows the determined LogD values of the ^{99m}Tc complexes in Na-phosphate, TRIS buffer and PBS.

**Table EX-3**

| Radioligand | LogD (shake-flask, octanol / *medium*) | | |
|---|---|---|---|
| | *Na-phosphate* ^{∗} *pH 7.4* | *TRIS buffer 0.1 M pH 7.4* | *PBS 0.1 M pH 7.4* |
| [^{99m}Tc]TcO-**1** [=[^{99m}Tc]TcO-ABX408] | - 2.18 ± 0.002 | - 2.18 ± 0.01 | - 1.62 ± 0.01 |
| [^{99m}Tc]TcO-**2** [= [^{99m}Tc]TcO-ABX451] | - 2.08 ± 0.01 | - 2.16 ± 0.02 | - 2.16 ± 0.03 |
| [^{99m}Tc]TcO-**3** [= [^{99m}Tc]TcO-ABX455] | - 1.93 ± 0.01 | - 2.08 ± 0.02 | - 2.07 ± 0.01 |
| [^{99m}Tc]TcO-**5** [=[^{99m}Tc]TcO-ABX474] | -2.41 ± 0.08 | - 1.98 ± 0.032 | - 2.52 ± 0.03 |

| | | | |
|---|---|---|---|
| ^{∗} 4.4 mM Na₂HPO₄, 2.2 mM NaH₂PO₄ | | | |

### Example 12

### Stability Studies

The stability of the complexes of ^{99m}Tc with the compounds 1 to 6 were determined in different media.

In brief, the freshly produced ^{99m}Tc-labeled radioligand solution (10-20 MBq, 20-100 µL) was added to the respective medium (200-400 µL), vortexed quickly, and shook gently at the respective temperature mentioned below. At certain time points, samples were taken, diluted, and measured by radio-RP-HPLC. Hereby, investigation of plasma samples included protein precipitation with the 4-fold volume of an ice-cold mixture of methanol/water (4/1, v/v), intensive shaking (5 min) and centrifugation (14,000 rpm, 10 min), before inspection of the supernatant. In addition, the recovery of extracted activity was calculated after activity measurement of supernatant and residue by a gamma counter.

**Table EX-4: Product stability at room temperature**

| Radioligand | Fraction of radioligand^{∗} at indicated time point after preparation (without addition of any media) | | | |
|---|---|---|---|---|
| | 0 h | 2 h | 4 h | 6 h |
| [^{99m}Tc]TcO-**3** [= [^{99m}Tc]TcO-ABX455] | 96% | 93 % | 92% | 91 % |
| [^{99m}Tc]TcO-**5** [= [^{99m}Tc]TcO-ABX474] | 94% | 91 % | 95% | 94% |
| [^{99m}Tc]TcO-**6** [=[^{99m}Tc]TcO-ABX490] | 89% | 87% | 88% | 85 % |

| | | | | |
|---|---|---|---|---|
| ^{∗} determined by radio-RP-HPLC, method a | | | | |

**Table EX-5: Product stability in DPBS and physiological saline**

| Radioligand | Fraction of radioligand^{∗} at indicated time point in DPBS^{#} at 25°C | | | |
|---|---|---|---|---|
| | 1 *h* | *2 h* | *4 h* | 20 *h* |
| [^{99m}Tc]TcO-**1** [=[^{99mT}c]TcO-ABX408] | 101.5 ± 0.6 % | 99.5 ± 0.8 % | 100.2 ± 1.0 % | 93.5 ± 1.8 % |
| [^{99m}Tc]TcO-**2** [= [^{99m}Tc]TcO-ABX451] | 89.9 ± 2.3 % | 97.7 ± 2.3 % | 80.7 ± 12.0 % | 89.2 ± 2.7 % |
| [^{99m}Tc]TcO-**3** [=[^{99mT}c]TcO-ABX455] | 98.2 ± 1.9 % | 97.3 ± 3.6 % | 98.4 ± 1.0 % | 89.1 ± 7.4 % |
| [^{99m}Tc]TcO-**4** [=[^{99mT}c]TcO-ABX456] | 88.4 ± 14.8 % | 100 ± 1.8 % | 105.5% | 98.0 ± 1.0 % |
| [^{99m}Tc]TcO-**5** [= [^{99m}Tc]TcO-ABX474] | 101.8 ± 0.03 % | 100.6 ± 0.3 % | 100.3 ± 1.0 % | 96.7 ± 2.1 % |
| [^{99m}Tc]TcO-**6** [=[^{99mT}c]TcO-ABX490] | 75.9 ± 0.2 % | 74.4 ± 0.1 % | 72.3 ± 0.9 %^{&} | 18.7 % |

| | | | | |
|---|---|---|---|---|
| ^{∗} indicated as mean ± SD (n= 2-4), determined by radio-RP-HPLC, method a) as described in Example 10, and normalized to the fraction of radioligand determined immediately after preparation ^{#} DPBS (Dulbecco's phosphate-buffered saline, modified: + Ca²⁺, +Mg²⁺) *^{&}* after 6 h instead of 4 h | | | | |

**Table EX-6: Product stability in mouse plasma (in vitro)**

| Radioligand | Fraction of radioligand^{∗} after 6 h in mouse plasma at 37°C |
|---|---|
| [^{99m}Tc]TcO-**1** [=[^{99m}Tc]TcO-ABX408] | 96.8 ± 0.3 % |
| [^{99m}Tc]TcO-**2** [= [^{99m}Tc]TcO-ABX451] | 98.3 ± 1.1 % |
| [^{99m}Tc]TcO-**3** [=[^{99m}Tc]TcO-ABX455] | 98.5 ± 0.8 % |
| [^{99m}Tc]TcO-**5** [= [^{99m}Tc]TcO-ABX474] | 96.7 ± 1.3 % |

| | |
|---|---|
| ^{∗} indicated as mean ± SD (n= 3-4), determined by radio-RP-HPLC, method a) as described in Example 10 and normalized to the fraction of radioligand determined immediately after preparation; Recovery of activity after plasma precipitation: 93.3 ± 0.6 % (mean ± SD; n= 9) | |

**Table EX-7: Product stability in human plasma (in vitro)**

| Radioligand | Fraction of radioligand^{∗} at indicated timepoint in human plasma at 37°C | | | | |
|---|---|---|---|---|---|
| | *0* h | 1 *h* | 2 *h* | 4 *h* | 6 h |
| [^{99m}Tc]TcO-5 [[=^{99m}Tc]TcO-ABX474] | 95.8 % | 91.1 ± 0.6 % | 91.2 ± 0.2 % | 91.1 ± 0.8 % | 91.7 ± 0.3 % |

| | | | | | |
|---|---|---|---|---|---|
| ^{∗} indicated as mean ± SD (n= 5), determined by radio-RP-HPLC, method a) as described in Example 10; Recovery of activity after plasma precipitation: 92.4% ± 0.9 (mean ± SD; n= 20) | | | | | |

### Example 11

### In vitro assays

Example 11 describes in vitro assays which were performed to determine the properties of the compounds **1, 2, 3, 4, 5** and **6.** For this reason, complexes of compound **1, 2, 3, 4, 5** or **6** with ^{99m}Tc as described in Example 10 and complexes of compound **1, 2, 3, 4, 5** or **6** with ^{nat}Re as described in Example 9 were prepared.

### a) Cell culture

Competition, saturation and internalization assays were assessed using the high PSMA expressing human prostate carcinoma cell line LNCaP (ATCC^{®} CRL-1740). The cells were grown as monolayer at 37°C in a humidified atmosphere comprising 5% CO2 and 95% air in RPMI medium including 10% FCS (Merck KGaA, Germany). After washing the confluent cells twice with phosphate buffered saline (PBS) and detaching them with trypsin/EDTA (0.05%/0.02%), the cells were suspended in medium and counted (Casy TT, Omni Life Science, Germany).

### b) ⁶⁸Ga-labeling of PSMA-11

A ⁶⁸Ga generator was purchased from iThemba LABS (Republic of South Africa). PSMA-11 (2-4 µg = 2.11-4.21 nmol) was labeled with ⁶⁸Ga (100 to 200 MBq) in a mixture of ammonium acetate (2 M) and HCl at a pH of 4.5. The reaction mixture was incubated for 10 min at 90°C. Quality control of radiolabeled PSMA-11 was performed using high-performance liquid chromatography (HPLC) with a C-18 reversed-phase column (semi-preparative Zorbax 300SB-C18, 9.4 × 250 mm 5 µm; Agilent Technologies, USA). The radiochemical yield was >97% for [⁶⁸Ga]Ga-PSMA-11 at molar activities between 30 and 60 GBq/µmol.

### c) Determination of the competitive and the direct binding affinity (Competition and Saturation)

For competition monolayer of LNCaP cells were seeded, 1 × 10⁵ cells/well in 24-well plates two days before the assay. On the day of competition, after aspirating the medium, 100 µL of PBS and, for competition 100 µL of different concentrations of the test compound in PBS (10⁻¹² to 10⁻⁶ M) were pipetted into the wells, simultaneously to 400 µL of medium including 1 nM of [⁶⁸Ga]Ga-PSMA-11.

For saturation monolayer of LNCaP cells were seeded, 5×10⁴ cells/well in 48-well plates two days before the assay. On the day of saturation, after aspirating the medium, 160 µL of medium (for ^{99m}Tc-labeled radioligand with D-mannitol (1 mg/mL)) were pipetted per well, for non-specific binding samples including 100 µM 2-PMPA. After preincubation for 5 min, 40 µL activity ([⁶⁸Ga]Ga-PSMA-11 or ^{99m}Tc-labeled radioligand solution) were added (total volume / well: 200 µL). Eight concentrations ranged between 0.3 and 40 nM.

After 1 hour of incubation at 37°C both for competition and saturation samples, the supernatants were aspirated and the cells washed twice with cold PBS. The cell lawn was lysed with 500 µL NaOH/SDS (0.1 M/1%) by shaking for 3 to 5 minutes. After transferring the lysates into measuring tubes, the activity of the samples was measured in a gamma counter (2480 Automatic Gamma Counter Wizard 2, Perkin Elmer, USA).

The half maximal inhibitory concentrations (IC₅₀) and the dissociations constants (K_{d}) were calculated by fitting the data using a nonlinear curve-fitting program (GraphPad Prism 9). With the K_{d} value of [⁶⁸Ga]Ga-PSMA-11 on LNCaP and the known concentration of [⁶⁸Ga]Ga-PSMA-11 in the competition assay the inhibition constants (Kᵢ) was also determined with the curve-fitting program. The Kᵢ values of the ^{nat}Re-complexes and the K_{d} values of [^{99m}Tc]TcO-complexes are shown in table EX-8.

**Table EX-8**

| compound | Ki[nM]^{∗} of ^{nat}Re-Complex | K_{d} [nM]^{∗} of [^{99m}Tc]TcO-complexes |
|---|---|---|
| **1** [ABX408] | 4.8 ± 3.4 | 15.5 ± 3.2 |
| **2** [ABX451] | n.d. | 32.1 ± 24.3 |
| **3** [ABX455] | 7.3 ± 1.6 | 15.8 ± 9.1 |
| **4** [ABX456] | n.d. | 4.7 ± 3.2 |
| **5** [ABX474] | 9.3 ± 0.9 | 7.2 ± 1.7 |
| **6** [ABX490] | n.d. | 7.3 |
| ^{nat}Ga-PSMA-11 | 8.5 ± 0.9 | 12.4 ± 2.1 |

| | | |
|---|---|---|
| ^{∗} mean ± SEM; n.d. = not determined | | |

### d) Determination of the internalization

For internalization LNCaP and PC3 cells were seeded, 1×10⁵ cells/well in 24-well plates two days before the assay. On the day of internalization, after aspirating the medium, 100 µL of PBS and, for non-specific binding samples, 100 µL 2-PMPA (100 µM) were pipetted into the wells, followed by 400 µL medium including activity. The concentration of the ^{99m}Tc-labeled radioligands was 25 nM. After 1 h of incubation at 37°C and adjacent well plates at 4°C, the supernatant was removed and the cells washed with cold PBS. Surface-bound activity was stripped with 4°C cold acid-wash buffer (0.2 M glycine, pH 2.8) for 5 min. The acid wash buffer was transferred from the plate wells to measuring tubes, as was the PBS buffer after washing once (binding on the surface). Cytosolic activity was determined after treatment with cell lysis buffer (0.1 M NaOH/1% SDS). Cell surface and cytosolic activity were measured separately in a gamma counter. Determination of protein content from cell lysate was performed using a spectrophotometer (NanoDrop, Thermo Fisher Scientific, USA) at an absorbance of 280 nm. The results are shown in Table EX-9.

**Table EX-9**

| %AD/mg Protein | [^{99m}Tc] TcO-3 [= [^{99m}Tc] TcO-ABX4551 | [^{99m}Tc]TcO-4 [^{99m}Tc]TcO - ABX456 | [^{99m}Tc] TcO-5 [= [^{99m}Tc]TcO -ABX474] | [^{99m}Tc] TcO-6 [= [^{99m}Tc]TcO -ABX490] | ⁶⁸Ga-PSMA11 |
|---|---|---|---|---|---|
| Binding^{∗}, 37°C | 1.8 ± 0.3 | 2.9 ± 0.5 | 3.2 ± 1.0 | 4.7 ± 0.2 | 0.8 ± 0.2 |
| Internalization^{∗}, 37°C | 1.0 ± 0.3 | 1.9 ± 0.9 | 2.8 ± 0.8 | 2.9 ± 0.2 | 0.5 ± 0.2 |
| Binding^{∗}, 4°C | 2.1 ± 1.2 | 2.2 ± 0.1 | 2.5 ± 0.3 | 2.5 ± 0.4 | 0.3 ± 0.4 |
| Internalization^{∗}, 4°C | 0.7 ± 0.2 | 0.2 ± 0.0 | 0.2 ± 0.0 | 0.2 ± 0.0 | 0.1 ± 0.0 |
| %Internalization, 37°C | 35.8 | 39.2 | 46.6 | 38.5 | 40.3 |

| | | | | | |
|---|---|---|---|---|---|
| ^{∗} mean ± SEM | | | | | |

Compared to comparative compound **3** the addition of the substituted or unsubstituted amino acids X and Y leads to an increase of the internalized %AD/mg protein.

### Example 12

### SPECT/CT and PET/CT Imaging

All animal experiments were carried out according to the guidelines of the German Regulations for Animal Welfare and have been approved by the local Ethical Committee for Animal Experiments.

A prostate cancer xenograft model was generated via subcutaneous injection of human LNCaP cells into the right shoulder of 8-12 week-old male nude mice (Rj:NMRI-*Foxn1*^{nu/nu}, Janvier Labs, Le Genest-Saint-Isle, France). Imaging studies were performed when tumors reached a diameter of > 6 mm. General anesthesia was induced and maintained with inhalation of 10% (v/v) desflurane in 30/10% (v/v) oxygen/air. During anesthesia, animals were continuously warmed at 37°C.

Small animal single-photon emission computed tomography (SPECT) was performed using the nanoSPECT/CT scanner (Mediso Medical Imaging Systems) equipped with an APT63 aperture consisting of four M3 multi-pinhole collimators. Each animal received 30 MBq of ^{99m}Tc-labeled compounds delivered in 0.2 mL of Dul-becco's phosphate-buffered saline as a single intravenous injection through a tail vein catheter. Photon emission was recorded using frame times of 60 s (1 h scan: 40-70 min), 90 s (4 h scan, 3.5-4.5 h), and 320 s (20 h scan, 18.5-21.5 h), and binned simultaneously within the 20% energy window of the 140.5 keV photopeak. With each SPECT scan, a corresponding CT image was recorded and used for anatomical referencing and attenuation correction. SPECT images were reconstructed using the Tera-Tomo^{™} three-dimensional (3D) algorithm at normal range with a voxel size of 0.4 mm and applying corrections for scatter attenuation, and decay.

Small animal positron emission tomography (PET) was performed using the nanoPET/CT scanner (Mediso Medical Imaging Systems). Each animal received 10 MBq of the radiolabeled reference compound [⁶⁸Ga]Ga-PSMA-11 delivered in Dul-becco's phosphate-buffered saline as a single intravenous injection through a tail vein catheter. Emission of the 511 keV annihilation photons was recorded continuously at a coincidence mode of 1:5 for 60 min following radiotracer injection. With each PET scan, a corresponding CT image was recorded and used for anatomical referencing and attenuation correction. Three-dimensional list mode data were binned using the 400-600 keV energy window. PET images of the 40-60 min time frame were reconstructed using the Tera-Tomo^{™} three-dimensional (3D) algorithm with a voxel size of 0.4 mm and applying corrections for random events, scatter, attenuation, and decay.

All images were post-processed and analyzed using ROVER (ABX) and displayed as maximum intensity projections at indicated scaling. Three-dimensional VOIs were created applying fixed threshold for delineation of tumor (30%), muscle (0%); and kidneys (39%). Standardized uptake values (SUV = [MBq detected activity/mL tissue] / [MBq injected activity/g body weight], mL/g) were determined and reported as max SUV (VOI-maximum). Time-activity curves were generated and further analyzed using Prism (GraphPad Software, San Diego CA, USA).

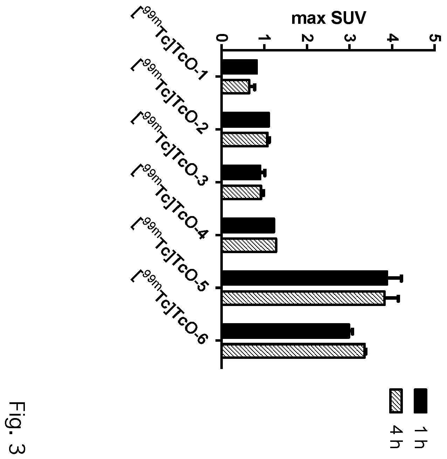

The tumor-uptake of [^{99m}Tc]-labeled radioligands in LNCaP tumor bearing mice is shown in Fig. 3 (SUV = standardized uptake value; error bars represent SEM of measured values). In contrast to internalization in cell culture (Example 11) no significant increase in tumor uptake for X and/or Y represented by phenylalanine or substituted phenylalanine could be seen. Surprisingly, for X and Y represented by glutamic acid a marked or significant increase in tumor uptake (highest uptake for A = A1, second highest for A = A2) has been found.

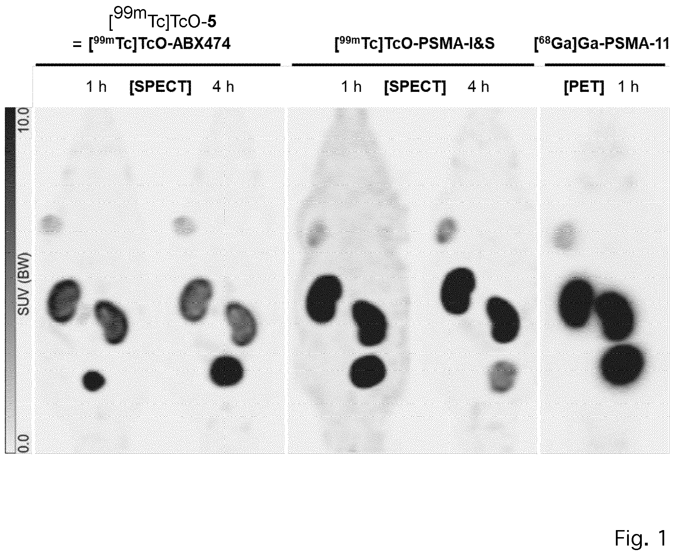

Fig. 1 shows the functional imaging of subcutaneous LNCaP tumor xenografts in mice using [^{99m}Tc]TcO-**5** [= [^{99m}Tc]TcO-ABX474] compared to reference compounds (maximum intensity projections; (SUV) standardized uptake value).

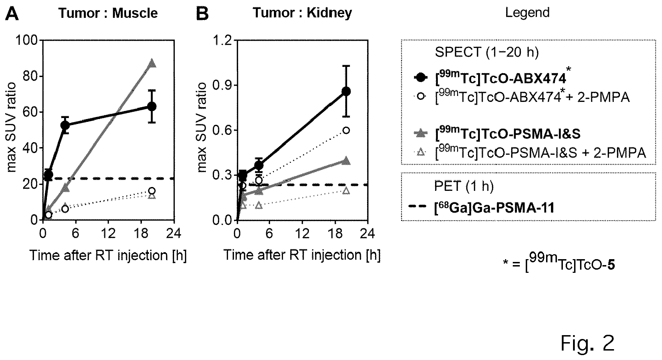

Fig. 2 shows tumor-to-background kinetics of [^{99m}Tc]TcO-**5** [= [^{99m}Tc]TcO-ABX474] compared to reference compounds in LNCaP tumor bearing mice ((SUV) standardized uptake value, (RT) radiotracer).

[^{99m}Tc]TcO-**5** [= [^{99m}Tc]TcO-ABX474] displayed similar uptake in tumors and lower uptake in kidneys compared to reference compounds along with a higher contrast compared to [*^{99m}*Tc]TcO-PSMA-I&S and higher image resolution compared to [⁶⁸Ga]Ga-PSMA-11 (see Fig. 1). Consequently, [^{99m}Tc]TcO-**5** [= [^{99m}Tc]TcO-ABX474] displayed higher tumor-to-muscle ratios and tumor-to-kidney ratios compared to reference compounds [*^{99m}*Tc]TcO-PSMA-I&S and [⁶⁸Ga]Ga-PSMA-11 within the initial 4 h after injection (see Fig. 2). These results indicate that [^{99m}Tc]TcO-**5** [= [^{99m}Tc]TcO-ABX-474] provides improved tumor visualization compared to the reference compounds. Blockade of radiotracer uptake in tumors through co-administration of 1.5 mg 2-PMPA confirmed PSMA-specific binding (see Fig. 2).

### List of abbreviations

- Ac: acetate
- ACN: acetonitrile
- AD: applied dose
- Boc: *tert*-butoxycarbonyl
- B0C₂O: di-*tert*-butyl dicarbonate
- Bp: boiling point
- CT: computed tomography
- 2-CTC: 2-chlorotrityl chloride
- DCM: dichloromethane
- Dde: *N*-(1-(4,4-dimethy1-2,6-dioxocyclohexylidene)ethyl)
- DIPEA: *N,N-*diisopropylethylamine
- DMF: dimethylformamide
- DPBS: Dulbecco's phosphate-buffered saline
- EDT: 1,2-ethanedithiol
- EDTA: ethylenediaminetetraacetic acid
- Et₂O: diethyl ether
- FCS: fetal calf serum
- Fmoc: fluorenylmethoxycarbonyl
- HATU: (1-[bis(dimethylamino)methylene]-1H-1,2,3-triazolo[4,5-b]pylidinium 3-oxide hexafluorophosphate
- HOAt: 1-hydroxy-7-azabenzotriazole
- HYNIC: hydrazinonicotinic acid
- IPA: 2-propanol
- LNCaP: Lymph Node Carcinoma of the Prostate, a human prostate cancer cell line
- logD: distribution coefficient
- NaOAc: sodium acetate
- NMM: 4-methylmorpholine
- NHS: *N*-hydroxysuccinimide
- PBS: phosphate buffered saline
- PC3: a human prostate cancer cell line
- PET: positron emission tomography
- 2-PMPA: 2-(phosphonomethyl)pentanedioic acid
- PSMA: prostate-specific membrane antigen
- PyBOP: (benzotriazol-1-yloxy)tripyrrolidinophosphonium hexafluorophosphate
- RLT: radioligand therapy
- r.t.: room temperature
- RP-HPLC: reversed-phase high-performance liquid chromatography
- RPMI: RPMI is a growth medium for cell culture
- RT: radiotracer
- SDS: sodium dodecyl sulfate
- SEM: standard error of the mean
- SPECT: single-photon emission computed tomography
- SPPS: solid phase peptide synthesis
- Sub: suberic acid
- SUV: standardized uptake value
- tBu: *tert-butyl*
- TFA: trifluoroacetic acid
- TFMSA: trifluoromethanesulfonic acid
- THF: tetrahydrofuran
- TIS: triisopropylsilane
- TRIS: tris(hydroxymethyl)aminomethane
- TSTU: *O*-(*N*-succinimidyl)-*N,N,N',N'*-tetramethyluronium tetrafluoroborate
- VOI: volume of interest

## Claims

1. A compound of general formula I 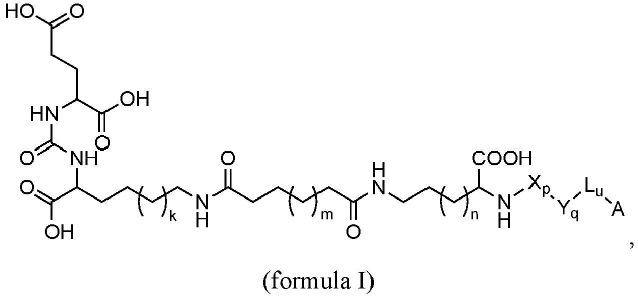 wherein
A is a chelator selected from the group consisting of 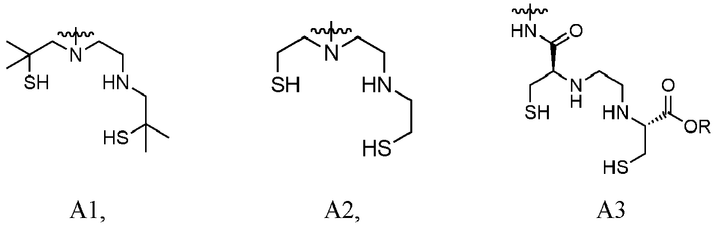 and 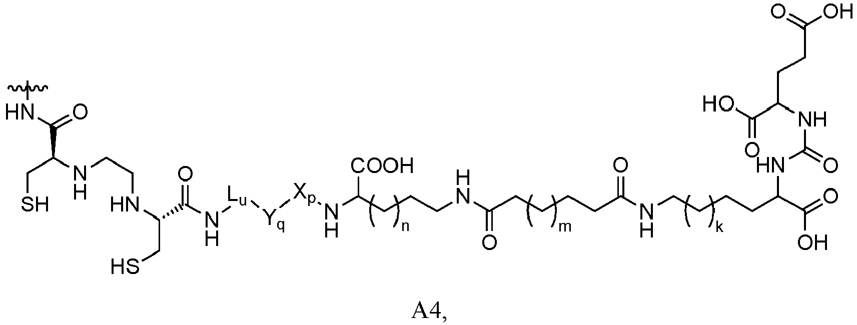
k is independently at each occurrence 0, 1, or 2;
m is independently at each occurrence 1, 2, 3, 4 or 5;
n is independently at each occurrence 0, 1, 2 or 3;
p is independently at each occurrence 1, 2 or 3;
q is independently at each occurrence 1, 2 or 3;
u is independently at each occurrence 0 or 1;
X and Y are substituted or unsubstituted amino acids;
L is a bifunctional linker selected from group, consisting of 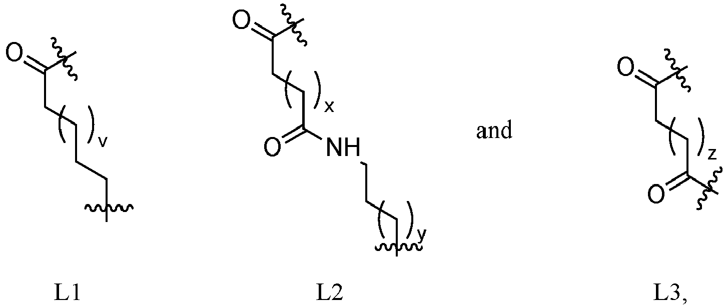
wherein v, x and y are independently of each other 0, 1, 2, or 3 and z is 0, 1, 2 ,3, 4 or 5; and
R is H, methyl or ethyl.

2. The compound of general formula I according to claim 1, wherein the amino acids are substituted or unsubstituted glutamic acid, substituted or unsubstituted aspartic acid, substituted or unsubstituted phenylalanine, substituted or unsubstituted histidine and substituted or unsubstituted serine.

3. The compound of general formula I according to claim 1 or claim 2, wherein the amino acids are substituted or unsubstituted phenylalanine or substituted or unsubstituted glutamic acid.

4. The compound of general formula I according to any one of the preceding claims, wherein L is L1 or L2.

5. The compound of general formula I according to any one of the preceding claims, wherein A is A1 or A2.

6. The compound of general formula I according to any one of the preceding claims, wherein k is 1, m is 3, n is 2, p is 1 or 2, q is 1 or 2 and u is 1, X and Y independently are substituted or unsubstituted phenylalanine or glutamic acid; L is L1 with v = 1 or L2 with x and y = 1, and A is A1 or A2.

7. The compound according to any one of the preceding claims, wherein the compound of general formula I is selected from the group consisting of

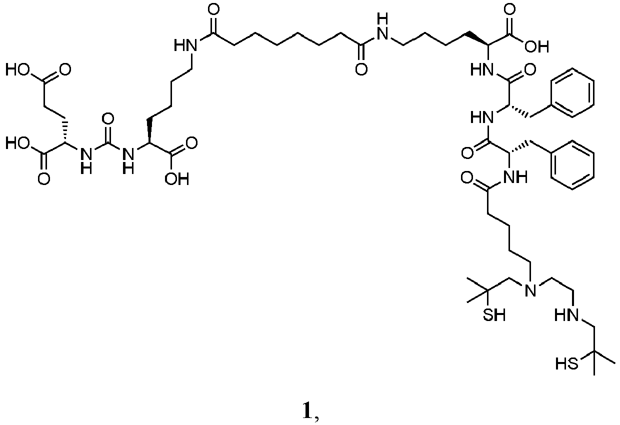

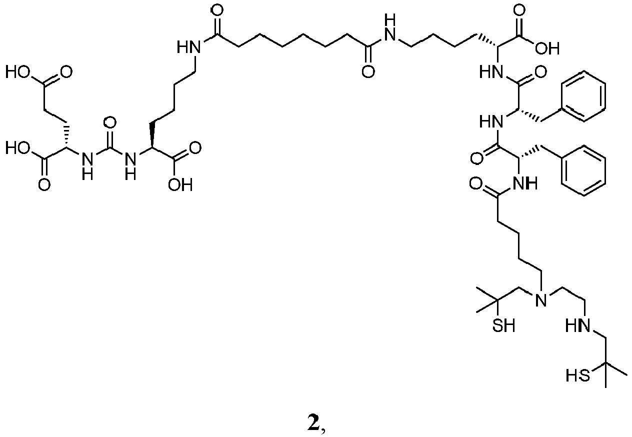

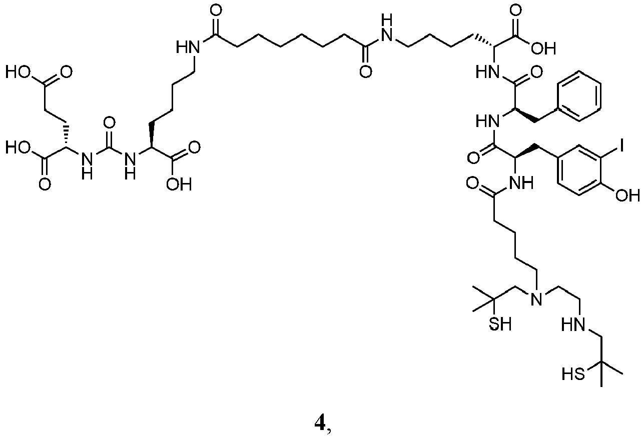

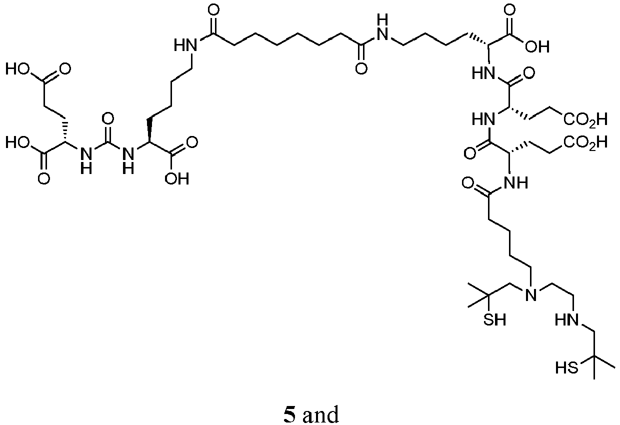

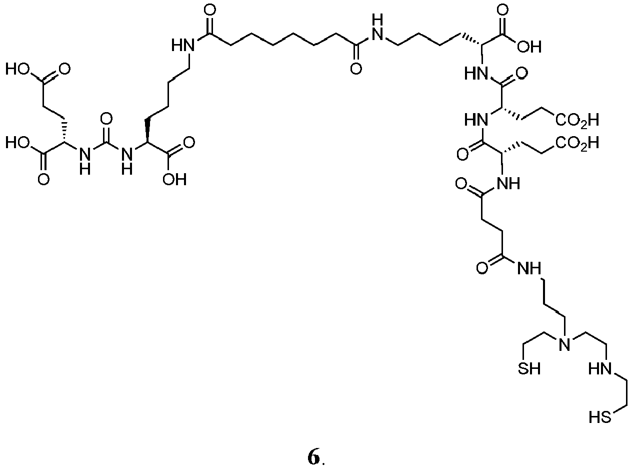

8. A complex of a compound according to any of claims 1 to 7 as ligands and a metal.

9. The complex according to claim 8, wherein the metal is an isotope selected from the group consisting of ^{99m}Tc, ⁹⁹Tc, ^{94m}Tc, ¹⁸⁶Re, ¹⁸⁸Re, ⁶⁴Cu and ⁶⁷CU.

10. A complex according to claim 8 or claim 9 for use as a medicinal drug.

11. A complex according to claim 8 or 9 for use as a medicinal drug for the diagnosis and treatment of diseases in which PSMA is involved.

12. The complex according to claim 10 or 11, **characterized in that** the medicinal drug is a radiopharmaceutical for nuclear medical imaging or radioligand therapy.

13. A medicinal drug containing a complex according to claim 8 or 9 or a pharmaceutically acceptable salt thereof.

14. A method for preparing a complex according to claims 8 or 9, wherein a compound according to any of claims 1 to 7 is contacted with the metal.

15. A method according to claim 14, wherein the compound is contacted with the metal at a reaction temperature in the range of from 20 to 100°C at ambient pressure.
